# EUROPEAN PATENT SPECIFICATION

(11) **EP 1 469 824 B1**
(45) Date of publication and mention of the grant of the patent: **07.09.2005**
(21) Application number: 02713167.1
(22) Date of filing: 22.03.2002
(51) Int. Cl.: A61K 9/00, A61K 31/00

(54) **PHARMACEUTICAL COMPOSITION FOR EXTENDED/SUSTAINED RELEASE OF THERAPEUTICALLY ACTIVE INGREDIENT**
PHARMAZEUTISCHE KOMPOSITION FÜR VERLÄNGERTE/LANGANHALTENDE FREISETZUNG EINES THERAPEUTISCH AKTIVEN WIRKSTOFFES
COMPOSITION PHARMACEUTIQUE POUR LA LIBERATION PROLONGEE/A EFFET RETARD D'UN PRINCIPE THERAPEUTIQUEMENT ACTIF

(30) Priority: 30.01.2002 IN DE20010096
(43) Date of publication of application: 27.10.2004
(73) Proprietor: COUNCIL OF SCIENTIFIC AND INDUSTRIAL RESEARCH, New Delhi 110 001 (IN)
(72) Inventor: GARG, Sanjay, c/oNIPER, 160 062 Punjab (IN); VERMA, Rajan, Kumar, c/oNIPER, 160 062 Punjab (IN); KAUL, Chaman, Lal, c/oNIPER, 160 062 Punjab (IN)
(74) Representative: Cabinet Hirsch
(86) International application number: PCT/IN2002/000062
(87) International publication number: WO 2003/063825

(56) References cited:
- EP-A- 0 309 051
- EP-B- 0 357 369
- WO-A-94/12152
- US-A- 4 326 525
- US-A- 5 612 059

## Description

### Technical Field

The present invention relates to a pharmaceutical composition for sustained/extended release of a therapeutically active moiety.

The invention pertains to both a useful and novel pharmaceutical composition for sustained release of therapeutically active ingredients to an environment of use. More specifically, the present invention relates to a pharmaceutical composition for oral use, which operates on the principles of osmotic pressure, diffusion, or a combination of both. The pharmaceutical composition, in the present invention, comprises of tablet core of a therapeutically active ingredient, solubility modifier, osmagents, and other conventional excipients. The tablet core is coated with a rate controlling membrane wall, made up of a semi-permeable and permeable membrane forming polymers. The therapeutically active ingredient, in the present invention, is weakly acidic in nature and is having a limited solubility in the aqueous environment. The solubility modifiers, which are present in the tablet core and are in immediate contact with the therapeutically active ingredient, are capable of improving the solubility of the said agent within the core, for example by changing the micro environmental pH. These agents improve the solubility of the therapeutically active ingredient within the pharmaceutical composition by elevating the micro environmental pH above the pKa of the therapeutically active ingredient and thus, improve its release profile from the pharmaceutical composition. Consequently, the release profile of the therapeutically active ingredient can be modulated and controlled by proper selection of the solubility modifying agents, based upon their ability to change the micro environmental pH. Thus, the release of the therapeutically active ingredient from the pharmaceutical composition will be independent of its intrinsic water solubility and the surrounding environment of use. In the present invention, solubility modulation of the therapeutically active ingredient, which is weakly acidic in nature, is achieved through the use of alkalinizing agents and/or buffers, which are in immediate contact with the therapeutically active ingredient and capable of elevating the micro environmental pH of the core above the pKa of the therapeutically active ingredient and thus improving its solubility.

### Background Art

Pharmaceutical compositions for extended delivery of drugs, to the environment of use, are well known in the prior art. Reference may be made to U.S. Pat. Nos. 3,845,770 and 3,916,889 wherein a semi-permeable wall surrounds an osmotically active drug core. The wall is permeable to water but is substantially impermeable to the components of the core. These tablets function by allowing fluids such as gastric or intestinal fluid to permeate the membrane and dissolve the active ingredient so it can be released through a passageway in the membrane. Generally, these pharmaceutical compositions are remarkably effective for delivering drugs that are soluble in the aqueous fluid and exhibit an osmotic pressure gradient across the wall against the fluid. However, it is difficult to deliver drugs, having limited solubility in the aqueous fluids, at meaningful and useful rates. Reference may also be made to U.S. Pat. No. 4,111,202, wherein the delivery kinetics of the drug, including those that are insoluble in aqueous fluids, was improved by manufacturing the pharmaceutical compositions having two compartments. An internal film, which was movable from a rested to an expanded state, separated the drug compartment and the osmotic compartment. Imbibition of the aqueous fluids, through the semi-permeable wall, to the lower osmotic compartment produces the solution that causes the lower compartment to increase in volume and act as a driving force that is applied against the film. This force causes the film to expand in the system against the drug compartment and, correspondingly, diminish the volume of the drug compartment, thereby delivering the drug through the passageway in the membrane. While this composition operates successfully for its intended use, and can deliver agents of varying solubility, its use can be limited because of the manufacturing steps and costs needed for fabricating and placing the movable film in the compartment of the osmotic system. U.S. Patent No. 4,327,725 describes an osmotic pharmaceutical composition wherein a semi-permeable wall surrounds a compartment containing a drug that is insoluble to very soluble in aqueous and biological fluids, and an expandable hydrogel. In operation, the hydrogel expands in the presence of external fluid that is imbibed into the system and the drug is dispensed through the passageway in the wall. This system operates successfully for its intended use but its use can be limited because the hydrogel can lack ability to imbibe sufficient fluid for the maximum self-expansion needed for dispensing the entire drug from the system. U.S. Patent No. 4,612,008 describes an osmotic pharmaceutical composition, wherein a semi-permeable wall surrounds the compartment comprising of a first osmotic composition comprising of a drug and an osmagent and a second composition containing a different osmotic agent and an osmopolymer. In operation, the lower compartment containing osmopolymers, swells after coming in contact with water and dispenses the drug from the drug compartment through the passageway in the wall. This composition works satisfactorily for delivering drugs having varying solubility but its use can be limited because of the manufacturing steps and costs needed for fabricating two compartments within the system. All the pharmaceutical compositions that have been discussed above involve a separate manufacturing step to create a orifice or an exit pore across the semi-permeable wall from where the drug is dispensed.

U.S. Patent No. 4,326,525 discloses an osmotic pharmaceutical composition using buffers, which reacts with the drug to produce a new compound having different thermodynamic properties from the parent drug. This composition is also based on semi-permeable membrane technology with a drilled hole acting as exit portal for the drug. U.S. Patent No. 5,284,662 describes osmotic composition for delivery of slightly soluble drugs. The composition consists of a water insoluble drug along with a swelling agent. This composition is also based on membrane wall technology with a drilled hole acting as a exit portal for the drug. U.S. Patent No. 4,755,180 describes an osmotic pharmaceutical composition; wherein the solubility of the drug is modulated by polymeric coated buffer components and osmagents. The drug and the release-modifying agent are coated separately by a rate controlling film and then mixed and compressed in the form of a tablet. The tablet core is further coated with a semi-permeable wall having a drilled hole. The dosage form works well with drugs having either high or low water solubility but the number of steps involved in the manufacturing are several and moreover, the control of solubility and thus the release of the drug depend mainly upon the release of the solubility-modifying agent from the coating, which itself can be affected by many factors and thus the drug may not be released at meaningful useful rates. U.S. Patent No. 5,736,159 describes a composition for controlled release of water insoluble drug. The composition consists of a core of water insoluble drug along with swellable polymers. The core is coated with a membrane wall, which does not have any preformed orifice for release of the drug. When the composition comes in contact with water, swelling of polymers in the core causes expansion of the partially hydrated core and a small opening forms at the weakest point of the membrane. Drug release takes place from this opening, which is at the edge of the tablet. This device can be used for delivery of water insoluble drugs but the release depends upon the formation of opening at the weakest point on the membrane, which can be quite variable depending upon the quality of the membrane. Moreover, the size of the opening is also difficult to control, which may result in variable drug release.

Pharmaceutical compositions for the controlled delivery of drugs to the environment of use, using microporous membrane, are also known to the prior art. U.S. Pat. No. 3,957,523 discloses a pharmaceutical composition, which has pH sensitive pore formers in the wall. When this composition is in the gastro intestinal tract, the pore former is partially or fully dissolved from the film by gastro-intestinal fluids to form a porous film. It is difficult to control the release from these systems because the selection of pore former is based on unknown acid and alkaline state of the gastro-intestinal tract, which concomitantly influences pore formation and exposure of drug to the fluid. The use of pore formers in substantially water impermeable polymers, such as polyvinyl chloride, is disclosed in J. Pharm. Sci., vol. 72, pp 772-775 and U.S. Pat. No. 4,244,941. The composition releases the core contents by simple diffusion through the pores in the coating and would be subject to environmental agitation. A similar kind of pharmaceutical composition is disclosed in U.S. Pat. No. 2,928,770, in which outer layer surrounding the drug consists of a porous material having its pores filled with a softened wax that is supposedly removed in the gastrointestinal tract by the fluids. This composition cannot be relied on for controlled release because it too requires in situ pore formation, which is dominated by unregulated external conditions and not by the composition. U.S. pat. No. 4,256,108, 4,160,452, and 4,200,098 discloses pharmaceutical compositions with pore formers in only one of at least two wall layers. These compositions contain a drilled hole through a semi-permeable coating for the release of the core contents. U.S. Pat. Nos. 4,880,631 and 4,968,507 discloses osmotic compositions coated with controlled porosity walls. The composition described in the above patents consists of a wall containing pH insensitive water-soluble additives, which after coming in contact with the aqueous fluids are leached into the surrounding environment leaving behind a microporous membrane. Though this type of composition is well suited for the delivery of drugs having high solubility, it has limited utility for delivering drugs having poor water solubility. Controlled porosity solubility-modulated pharmaceutical compositions for delivery of drugs having either high or low water solubility are described in U.S. Pat. Nos. 4,946,686 and 4,994,273. The composition described consists of controlled release solubility modulating agents, which are either surfactants or complexing agents and are either surrounded by a rate controlling membrane or dispersed in a matrix. However, the control of solubility and thus the release of the drug from these composition depend mainly upon the release of the solubility-modifying agent from the coating or the matrix, which itself can be affected by many factors and thus the drug may not be released at meaningful useful rates. Moreover, these compositions are well suited for delivery of drugs that do not have appreciable acid base character.

The use of asymmetric membranes in osmotic drug delivery has been disclosed in U.S. Pat. Nos. 5,612,059 and 5,698,220. The composition in these patents consists of tablet core surrounded by asymmetric membrane. These asymmetric membranes consist of a very thin, dense skin structure supported by a thicker, porous substructural layer. U.S. Pat. No. 5,697,922 describes capsule device coated with asymmetric membranes. These capsule devices consist of a poorly water-soluble drug along with solubility modifiers. The solubility modifier is in the form of mini-tablets, which is coated with a rate controlling membrane from where release of the solubility modifier takes place. Thus, the manufacturing step of the above device includes number of complicated manufacturing steps including compression of the solubility modifier and its coating with a rate controlling membrane. Also, the solubility and thus, the release of the drug can be affected by the release of solubility modifier from the coated tablets.

It will be readily appreciated by those versed in the subject art that though there are reports of using the pharmaceutical compositions for delivery of water-soluble drugs, very few reports are there for delivery of drugs having limited water solubility. In those cases where the compositions have been used for delivery of drugs having limited water solubility, the use of such compositions can be limiting because of the number of manufacturing steps involved in separately coating or dispersing the solubility modifier in a matrix. Moreover, the control of solubility and thus the drug release from these composition depend mainly upon the release of the solubility-modifying agent from the coating or the matrix, which itself can be quite variable and affected by many factors and thus the drug may not be released at meaningful useful rates. It will be further appreciated by those versed in the subject art that in majority of the pharmaceutical compositions mentioned in the prior art, complex manufacturing steps are involved either to make two compartments within the phamaceutical composition and/or to create a delivery orifice across the membrane wall from where the drug is released.

In the light of the above discussion, it will be readily appreciated by those versed in the subject art that a critical need exists for a pharmaceutical composition useful for extended release of therapeutically active ingredients that shows limited solubility in the aqueous or biological fluids. Likewise, it will be further appreciated by those skilled in the art, that there is a critical need for a pharmaceutical composition, which is simple in design, manufactured using less number of steps, and easily amenable to mass production. The usefulness of the composition will be further increased if the delivery of therapeutically active ingredient from such composition is not affected by its intrinsic solubility and the release properties of the solubility-modifying agent.

### Objects of the Invention

The main object of the present invention is to provide a pharmaceutical composition for sustained/ extended release of a therapeutically active ingredient, which obviates the drawbacks as detailed above.

Another object of the present invention is to provide a pharmaceutical composition for sustained release of a therapeutically active ingredient, said ingredient being weakly acidic in nature and having a pKa between 2.5 to 7.5 and having a limited solubility in the aqueous and biological fluids.

Still another object of the present invention is to provide a pharmaceutical composition that comprises of alkalinizing agents and/or buffers that are in immediate contact with the therapeutically active ingredient and are capable of elevating the micro environmental pH of the core above the pKa of the therapeutically active ingredient, thereby improving its solubility and release profile from the pharmaceutical composition.

Yet another object of the present invention is to provide a pharmaceutical composition that further comprises of osmotically effective solutes or osmagents that are soluble in water and capable of exhibiting an osmotic pressure gradient across the wall against the external fluids.

Another object of the present invention is to provide a pharmaceutical composition, which composition comprises of a rate controlling membrane consisting of semi-permeable and permeable membrane polymers that surrounds the tablet core compartment consisting of a poorly soluble weakly acidic therapeutically active ingredient along with alkalinizing agents and/or buffers capable of modulating the micro environmental pH, and osmagents.

Yet another object of the invention is to provide a pharmaceutical composition comprising of a rate controlling membrane that surrounds the core compartment, which rate controlling membrane comprises of a semi-permeable membrane polymer, which is water insoluble but permeable to the aqueous fluids and substantially impermeable to the components of the core, and a permeable membrane polymer, which is water soluble and permeable to aqueous fluids and at least one of the components of the core.

Still another object of the invention is to provide a pharmaceutical composition comprising of a rate controlling membrane surrounding the core compartment, where the rate controlling membrane comprises of a semi-permeable membrane polymer, permeable membrane polymer, and at least one plasticizer capable of modulating the film formation properties of the polymers.

Yet another object of the present invention is to provide a pharmaceutical composition, from where the drug release occurs through the mechanisms of osmotic pumping, diffusion, or a combination of both and is simple in design and amenable to mass production.

Other objects, features and advantages of the invention will be more apparent to those versed in the dispensing art from reading the detailed description of the specification, taken in conjunction with the drawing figures and accompanying claims.

### Summary of the invention

Accordingly, the present invention provides a pharmaceutical formulation comprising a tablet core and the said tablet core surrounded with polymer membrane for extended release of therapeutically active ingredients.

The present invention also provides a pharmaceutical composition for sustained release of therapeutically active ingredients to an environment of use. More specifically, the present invention relates to a pharmaceutical composition for oral use, which operates on the principles of osmotic pressure, diffusion, or a combination of both. The pharmaceutical composition, in the present invention, comprises of tablet core of a therapeutically active ingredient, solubility modifier, osmagents, and other conventional excipients. The tablet core is coated with a rate controlling membrane wall, made up of a semi-permeable and permeable membrane polymers

### Novelty of the invention

The novelty in the present invention is that a pharmaceutical composition, consisting of a weakly acidic therapeutically active ingredient having a limited solubility in the aqueous and biological fluids; alkalinizing agents and/or buffers that are in immediate contact with the therapeutically active ingredient and capable of elevating the micro environmental pH of the core above the pKa of the therapeutically active ingredient; osmagent; and other tableting excipients is prepared and coated with a membrane wall comprising of a water insoluble semi-permeable membrane forming polymer, water-soluble polymer, and plasticizer(s). Unlike the prior art, the solubility modifier, in the present invention, is in intimate contact with the therapeutically active ingredient. After coming in contact with the aqueous fluids, it dissolves readily and elevates the micro environmental pH of the tablet core above the pKa of the therapeutically active ingredient thus increasing the solubility of the therapeutically active ingredient. Thus, solubility modulation and release of the therapeutically active ingredient is not dependent upon the release of solubility modulating agent. Moreover, the ratio of water insoluble semi-permeable membrane forming polymer and water-soluble polymer can be adjusted to modulate the drug release from the composition, thereby avoiding the need to create a delivery orifice using a separate manufacturing step. Thus, the pharmaceutical composition, in the present invention, is simple in design, easy to manufacture, and easily amenable to mass production as compared to prior art and yet effective in extended release of drugs.

### Brief description of the accompanying drawings

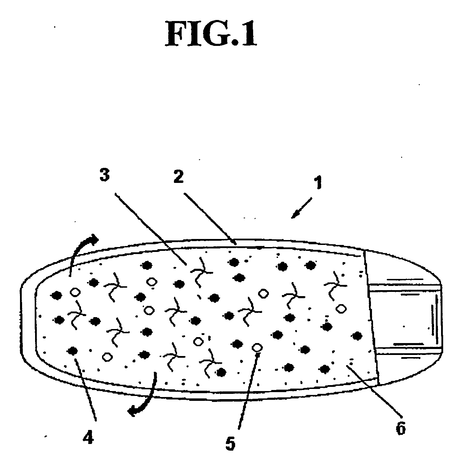

In the drawings accompanying this specification, **figure 1** represents one of the embodiment of the instant invention, wherein the pharmaceutical composition, 1, has a core compartment comprising of a therapeutically active ingredient, 3, alkalinizing agent(s)/buffer(s), 4, osmagent, 5, and other excipients, 6, as needed to form a tablet suitable for the application of a rate controlling membrane, 2, comprising of a semi-permeable and permeable membrane forming polymers. The alkalinizing agent(s)/buffer(s), 4, are in immediate contact with the therapeutically active ingredient, 3, and, after coming in contact with the aqueous fluids, are capable of elevating the micro environmental pH of the core above the pKa of the therapeutically active ingredient. In operation, aqueous fluids permeates wall 2 in response to the concentration and osmotic gradient of the core at a rate controlled by the permeability of the wall, entering the core compartment where the therapeutically active ingredient and excipients dissolve. Dissolution of alkalinizing agent/buffer results in the elevation of micro environmental pH of core above the pKa of the therapeutically active ingredient, thereby increasing its solubility. The dissolved therapeutically active ingredient and other excipients then exit through the membrane wall in response to the osmotic and concentration gradient.
**Figure 2** represents release profile of glipizide from compositions made in example 1 showing the effect of solubility modifier (alkalinizing agent) in comparison with the marketed extended release formulation. Also shown is the release profile from the composition after 3 months of storage at 40 °C and 75% relative humidity.
**Figure 3** represents release profile of glipizide from compositions made in example 2 showing the effect of varying the weight gain of the membrane wall.
**Figure 4** represents release profile of glipizide from compositions made in example 3 showing the effect of varying the concentrations of a water-soluble plasticizer (PEG-400) and plasticizer having limited solubility in water (Triacetin).
**Figure 5** represents release profile of glipizide from compositions made in example 4 showing the effect of varying the concentrations of water-soluble polymer (PVP).
**Figure 6** represents release profile of glipizide from composition made in example 5 showing the use of ethyl cellulose as a semi-permeable membrane forming polymer.
**Figure 7** represents release profile of glipizide from compositions made in example 6 showing the use of hydroxypropylmethyl cellulose as a water-soluble polymer.
**Figure 8** represents release profile of aspirin from compositions made in example 7 showing the effect of different concentrations of solubility modifier (alkalinizing agent).
**Figure 9** represents release profile of gliclazide from compositions made in example 8 showing the effect of different concentrations of solubility modifier (alkalinizing agent).

### Detailed description of the invention

Accordingly, the present invention provides a pharmaceutical composition for sustained release of a therapeutically active moiety, said composition comprising a tablet core surrounded by a release rate controlling membrane, said tablet core consisting of:
(i) the therapeutically active moiety having limited solubility in the aqueous fluids, weakly acidic in nature and having a pKa between 2.5 to 7.5,
(ii) an alkalinizing agent or a buffer compound in immediate contact with the above said therapeutically active moiety,
(iii) an osmotically effective solute that is soluble in water and capable of exhibiting an osmotic pressure gradient across the release rate controlling membrane against the external fluids, and
(iv) optionally containing one or more pharmaceutically acceptable excipients and polymers, said release rate controlling membrane consisting of:

(i) a semi-permeable membrane forming polymer which is insoluble in water but partially permeable to aqueous fluids and substantially impermeable to the core composition,
(ii) a permeable membrane forming polymer which is soluble in water and permeable to aqueous fluids, and to at least one of the moiety of the core composition, and
(iii) at least one plasticizer ranging between 2 to 60 % by weight, based on the - total weight of dry polymers.

One embodiment of the invention relates to the therapeutically active moiety which is a drug that acts on peripheral nerves, adrenergic receptors, cholinergic receptors, nervous system, skeletal muscles, cardiovascular, smooth muscles, blood circulatory system, synaptic sites, neuroeffector junctional sites, endocrine and hormone system, immunological system, reproductive system, skeletal system, autocoid systems, alimentary and excretory systems, inhibitory or autocoids and histamine systems, and those materials that act on the central nervous system such as hypnotics and sedatives.

Another embodiment, the therapeutically active moiety is selected from a group consisting of acetazolamide, acetyl salicylic acid, p-amino salicylic acid, captropil, carbenicillin, carbenoxolone, chlorpropamide, clofibrate, diclofenac, diflunisal, ethacrynic acid, etodolac, fenoprofen, furosemide, gliclazide, glimepiride, glipizide, glyburide, ibuprofen, indomethacin, ketoprofen, naproxen, nimesulide, tolazamide, tolbutamide, tolmentin and zomepirac and preferably selected from hypoglycemic agent and anti-inflammatory agent.

Still another embodiment, the hypoglycemic agent used is selected from the category of sulfonylurea consisting of glipizide, gliclazide, glimepiride, and glyburide.

Still another embodiment, the anti-inflammatory agent is selected from group consisting of aspirin, paracetamol, ibuprofen, indomethacin, ketoprofen, naproxen, nimesulide, tolmentin and zomepirac preferably selected from Aspirin.

Yet another embodiment, the dose of the therapeutically active moiety per tablet ranges between 0.1 mg to 600 mg.

Still yet another embodiment, the alkalinizing agent or the buffer used is soluble in water and improves the solubility of therapeutically active moiety in the aqueous fluids by increasing the micro environmental pH of the core above the pKa value of the therapeutically active moiety.

Still yet another embodiment, the alkalinizing agent used is selected from the group consisting of sodium bicarbonate, potassium bicarbonate, sodium citrate, potassium citrate, tris(hydroxymethyl)aminomethane, meglumine, and/or the mixture thereof and the buffer used is selected from the group consisting of sodium phosphates, potassium phosphates, sodium citrate, potassium citrate, sodium acetate, tris(hydroxymethyl)aminomethane, and/or mixtures thereof.

Still another embodiment, the ratio of the therapeutically active ingredient to the alkalinizing agent is in the range of 0.1:9.9 to 7:3.

Still another embodiment, the ratio of the therapeutically active ingredient to the buffer is in the range of 0.1:9.9 to 7:3.

Still yet another embodiment, the osmotically effective solute used is selected from the group consisting of sodium chloride, potassium chloride, mannitol, sorbitol, and carbohydrates selected from the group consisting of sucrose, glucose, fructose, dextrose, lactose, and mixtures thereof, and preferably selected from the group consisting of sodium chloride, mannitol, and lactose.

Still yet another embodiment, the core components used exerts osmotic gradient across the wall of release rate controlling membrane against the external fluids.

Still yet another embodiment, the semi permeable membrane-forming polymer is water insoluble allows water to permeate and substantially prevents permeability of compositions of the tablet core.

Still yet another embodiment, the semi-permeable membrane forming polymer is selected from group consisting of cellulose acetate, cellulose acetate butyrate, cellulose acetate propionate, ethyl cellulose, polymers of acrylic and methacrylic acid and esters thereof, preferably selected from cellulose acetate and ethyl cellulose.

Still yet another embodiment, the permeable membrane forming is a water-soluble allows water to permeate along with at least one of the components of the core.

Still yet another embodiment, the permeable membrane forming polymer is selected from the group consisting of polyvinyl alcohol, polyvinyl pyrrolidone, cellulose ethers, polyethylene glycols, polymers of acrylic and methacrylic acid and esters thereof, preferably selected from polyvinyl pyrrolidone and hydroxypropylmethyl cellulose.

Still yet another embodiment, the ratio of semi-permeable water insoluble polymer membrane to permeable water-soluble polymer membrane is in the range of 9:1 to 1:9, preferably in the range of 9:1 to 3:7.

Still yet another embodiment, the plasticizer used is a mixture of water-soluble and partially water-soluble plasticizer.

Still yet another embodiment, wherein the partially water soluble plasticizer used is selected from the group consisting of dibutyl sebacate, diethyl phthalate, dibutyl phthalate, triacetin, triethyl citrate, tributyl citrate and castor oil.

Still yet another embodiment, the water-soluble plasticizer used is selected from the group consisting of propylene glycol, glycerol, polyethylene glycols, liquid sorbitol, and/or mixture thereof.

Still yet another embodiment, the thickness of the membrane wall ranges between 1 to 1000 microns, preferably ranges between 50 to 500 microns.

Still yet another embodiment, the said composition optionally consists of one or more pharmaceutically acceptable excipients.

Still yet another embodiment relates to the mechanism of release of the therapeutically active ingredient which is based on combination of osmotic pumping and diffusion.

One more embodiment of the invention relates to a process for the preparation of pharmaceutical composition for sustained release of a therapeutically active moiety, said process comprising:
a. preparing a core composition by dry blending a therapeutically active moiety, an alkalinizing agent or a buffer compound, an osmotically effective solute and optionally containing one or more pharmaceutically acceptable excipients and polymers, or
b. preparing the core composition by slugging or wet granulation techniques by blending the drug with the other excipients including alkalinizing agent(s)/buffer(s) and osmagent using water, alcohol, or an organic co solvent such as isopropyl alcohol/methylene chloride, in the ratio 80/20, V/V as the granulation fluid to obtain wet granules, or
c. by dissolving the drug and the solubility modifier in a common aqueous or organic solvent and after evaporation to dryness, mixing the residue with osmagent and other excipients needed to obtain core composition,
d. compressing the above core composition obtained in steps (a), (b) and (c) to obtain tablets core by using conventional tablet making machine,
e. preparing coating solution by dissolving required amount of semi permeable membrane forming polymer in a solvent selected from methylene chloride, methanol, ethanol or mixture thereof,
f. adding permeable membrane forming polymer and one or more plasticizer to the solution of step (e) with continuous stirring,
g. coating the compressed tablet of step (d) with the coating solution of step (f) by using the techniques selected from press coating, spraying, dipping, or air suspension techniques and
h. drying the coated tablet core obtained in the step (g) at 45-60°C for about 16 hours to obtain the composition for sustained release and packing the tablets by conventional methods.

Another embodiment of the invention relates to a process, mixture obtained by dry blending in step (a) is passed through standard sieves to obtain uniform particle size to form core composition

Still another embodiment of the invention, the wet granules obtained in step (b) is dried at a temperature ranging between 40 and 60°C for about 10 minutes, passing the granules through 20-22 standard sieves to break agglomerates and to making it uniform particle size to obtain core composition

Another embodiment of the invention relates to the preparation of coating solution which surrounds tablet core by using the mixture of water insoluble semi-permeable membrane forming polymer and water-soluble polymer and it is possible to control the permeability of the membrane by varying the ratio of semi-permeable and permeable membrane forming polymers.

In general, increasing the concentration of water-insoluble semi-permeable membrane forming polymer will decrease the membrane permeability and the drug release. On the other hand, increasing the concentration of water-soluble polymer will increase the membrane permeability and the drug release. In operation, the core compartment imbibes aqueous fluids from the surrounding environment across the rate controlling membrane. Dissolution of the alkalinizing agent(s)/buffer(s), which are in immediate contact with the therapeutically active ingredient, results in the elevation of the micro environmental pH of the core above the pKa of the therapeutically active ingredient. The solubility of the therapeutically active ingredient and, thus, its release from the pharmaceutical composition is improved by the change in the micro environmental pH. By adjusting the amount and/or type of alkalinizing agent(s)/buffer(s), based upon their ability to modulate the micro environmental pH, the release profile of the drug can be adjusted to meet the desired kinetic profile.

In another embodiment of the invention provides pharmaceutical composition for extended release of a therapeutically active ingredient, which comprises
A. a tablet core comprising
   i) a therapeutically active ingredient having limited solubility in the aqueous fluids, and the said ingredient being weakly acidic in nature and having a pKa between 2.5 to 7.5, and
   ii) an alkalinizing agent or a buffer compound in immediate contact with the above said therapeutically active ingredient, and
   iii) an osmotically effective solute that is soluble in water and capable of exhibiting an osmotic pressure gradient across the wall against the external fluids; and
B. the said tablet core being surrounded by a membrane wall formed by a coating composition comprising
   i) a semi-permeable membrane forming polymer that is water insoluble but, at least in part, permeable to water and substantially impermeable to the core components and a polymer material that is soluble in water and permeable to water and to at least one of the components of the core and the ratio between the water insoluble polymer to water-soluble polymer ranging from 9:1 to 1:9, and
   ii) at least one plasticizer ranging between 2 to 60 % by weight, based on the total weight of dry polymers

In an embodiment of the present invention, pharmaceutical composition consists of a tablet core surrounded by a rate controlling membrane. The tablet core consists of a therapeutically active ingredient that is weakly acidic in nature and having a pKa between 2.5 to 7.5. The therapeutically active ingredient is having a limited solubility in the aqueous and biological fluids. The poor solubility of the therapeutically active ingredient is improved through the use of alkalinizing agent(s)/buffer(s), which are in its immediate contact. These alkalinizing agent(s)/buffer(s) are soluble in water and capable of elevating the micro environmental pH of the core above the pKa of the therapeutically active ingredient thereby improving its solubility. The core compartment also consists of osmotically effective agents or osmagents that are soluble in water and capable of exhibiting an osmotic pressure gradient across the wall against the external fluids. Therapeutically active ingredient, alkalinizing agent(s)/buffers, and osmagents may be combined with other conventional excipients as needed to from a core compartment of the delivery system. The core compartment is surrounded by a rate controlling membrane that consists of water insoluble semi-permeable membrane forming polymer, water-soluble permeable membrane forming polymer, and at least one plasticizer that is capable of improving film formation properties of the polymers. The semi-permeable membrane forming polymer is water insoluble but, at least in part, permeable to aqueous fluids and substantially impermeable to the components of the core. The permeable membrane forming polymer is water soluble and permeable to aqueous fluids and at least one of the components of the core. Optionally, the permeable membrane forming polymer dissolves in water resulting in formation of channels in the membrane, which permit the egress of drug and other excipients from the core in aqueous environment. In operation, the core compartment imbibes aqueous fluids from the surrounding environment across the rate controlling membrane. Dissolution of the alkalinizing agent(s)/buffer(s), which are in immediate contact with the therapeutically active ingredient, results in the elevation of the micro environmental pH of the core above the pKa of the therapeutically active ingredient. The solubility of the therapeutically active ingredient and, thus, its release from the pharmaceutical composition is improved by the change in the micro environmental pH. The alkalinizing agent or the buffer used can be selected based upon its ability to elevate the micro environmental pH of the core above the pKa of the therapeutically active ingredient and thus, improve its solubility and release from the pharmaceutical composition. By adjusting the amount and/or type of alkalinizing agent(s)/buffer(s), based upon their ability to modulate the micro environmental pH of the tablet core, the release profile of the therapeutically active ingredient can be adjusted to meet the desired kinetic profile. The dissolved therapeutically active ingredient is released across the rate controlling membrane, permeability of which can be modulated by proper choice of polymers and plasticizers and by varying the ratio of water insoluble semi-permeable membrane forming polymer to water-soluble polymer. The thickness of the rate-controlling membrane wall is directly proportional to the weight gain of the coating solution on the tablet cores. By adjusting the weight gain of the membrane wall on the tablet cores, thickness of the membrane wall can be controlled.

In the specifications and the accompanying claims, the term therapeutically active ingredient or a drug includes any physiologically or pharmacologically active substances that produce a localized or systemic effect or effects in animals, which term includes mammals, humans, and primates. The term also includes domestic household, sport or farm animals such as sheep, goats, cattle, horses, and pigs for administering to laboratory animals such as mice, rats and guinea pigs, and to fish to avians, to reptiles and zoo animals. The drug that can be delivered includes those drugs that act on peripheral nerves, adrenergic receptors, cholinergic receptors, nervous system, skeletal muscles, cardiovascular, smooth muscles, blood circulatory system, synaptic sites, neuroeffector junctional sites, endocrine and hormone system, immunological system, reproductive system, skeletal system, autocoid systems, alimentary and excretory systems, inhibitory or autocoids and histamine systems, and those materials that act on the central nervous system such as hypnotics and sedatives. Examples of the drug are disclosed in *Remington: The Science and Practice of Pharmacy,* vol. 1 and 2, 19^{th} Ed., 1995, published by Mack Publishing Co., Easton, PA; and in the *The Pharmacological Basis of Therapeutics,* by Goodman and Gilman, 9^{th} Ed., 1996, published by McGraw Hill Company, N.Y.; and *The Merck Index,* 12^{th} Ed., 1996, published by Merck & Co., N.J. Specific examples of therapeutically active ingredients that can be adapted for use in the present invention may include acetazolamide, acetyl salicylic acid, p-amino salicylic acid, captropil, carbenicillin, carbenoxolone, chlorpropamide, clofibrate, diclofenac, diflunisal, ethacrynic acid, etodolac, fenoprofen, furosemide, gliclazide, glimepiride, glipizide, glyburide, ibuprofen, indomethacin, ketoprofen, naproxen, nimesulide, tolazamide, tolbutamide, tolmentin, zomepirac.

The solubility-modifying agent in the present invention is alkaline in nature or it can be a buffer that is capable of elevating the micro environmental pH of the core above the pKa of the therapeutically active ingredient and thus is capable of increasing its solubility. The selected agent can act both as an alkalinizing agent or a buffer depending upon its property to elevate and maintain the pH. The criteria for selecting the alkalinizing agent or the buffer are based upon its ability to elevate the micro environmental pH of the core above the pKa of the therapeutically active ingredient. In the specifications and the accompanying claims, the term alkalinizing agent includes any agent(s) that is capable of elevating the micro environmental pH of the tablet core towards the alkaline side. Exemplary alkalinizing agents may include sodium hydroxide, potassium hydroxide, sodium carbonate, potassium carbonate, sodium bicarbonate, potassium bicarbonate, sodium tetraborate, sodium citrate, potassium citrate, potassium gluconate, sodium sulfite, dibasic ammonium phosphate, magnesium oxide, magnesium hydroxide, tris(hydroxymethyl)aminomethane, monoethanolamine, diethanolamine, meglumine, arginine, and the mixture of above. In the specifications and the accompanying claims, the term buffer includes any agent(s) that, by their presence in solution, resist changes in pH upon addition of small quantities of acid or alkali. The said buffer is able to elevate the micro environmental pH of the core above the pKa of the therapeutically active ingredient. Exemplary buffer compounds may include sodium phosphates, potassium phosphates, sodium citrate, potassium citrate, sodium acetate, tris(hydroxymethyl)aminomethane, monoethanolamine, diethanolamine, and the mixture of above.

There are a variety of pharmaceutical compositions that incorporates osmotically effective solutes in the device core. These agents are capable of causing an osmotic pressure gradient across the device wall and imbibe fluid into the device. The osmotically effective compound, also known as osmagent, which can be used in the present invention may include organic and inorganic compounds or solutes that exhibit an osmotic pressure gradient across the membrane, when placed in an aqueous environment. Osmotically effective compounds useful for this purpose may include magnesium sulfate, magnesium chloride, sodium chloride, lithium chloride, potassium sulfate, sodium carbonate, sodium sulfite, lithium sulfate, potassium chloride, calcium bicarbonate, sodium sulfate, calcium sulfate, potassium acid phosphate, calcium lactate, mannitol, urea, inositol, sorbitol, magnesium succinate, tartaric acid, carbohydrates such as raffinose, sucrose, glucose, fructose, dextrose, lactose, and mixtures of above said osmagents.

The semi-permeable membrane forming polymer, in the present invention, is water insoluble but, at least in part, permeable to aqueous fluids and substantially impermeable to the components of the core. Materials that can be used to form the semi-permeable membrane may include cellulose esters such as cellulose acetate, cellulose acetate butyrate, cellulose acetate propionate, cellulose diacetate, cellulose triacetate; cellulose ethers such as ethyl cellulose; polyvinyl acetates, polyesters, polyethylene, ethylene vinyl alcohol copolymer, polypropylene, polyvinyl chloride, polyurethane, polycarbonate, polymers of acrylic and methacrylic acid and esters thereof, and mixtures of above said polymers.

The permeable membrane forming polymer, in the present invention, is water soluble and permeable to aqueous fluids and at least one of the components of the core. Optionally, the permeable membrane-forming polymer dissolves in water resulting in formation of channels in the membrane, which permit the egress of drug and other excipients from the core in aqueous environment. Materials used to form the permeable membrane may include polyvinyl alcohol, polyvinyl pyrrolidone, alkyl and hydroxyalkyl celluloses such as methyl cellulose, hydroxypropylmethyl cellulose, hydroxybutylmethyl cellulose, hydroxypropyl cellulose, sodium carboxy methyl cellulose, hydroxyethylmethyl cellulose, and hydroxyethyl cellulose; cellulose acetate phthalate, hydroxypropylmethyl cellulose phthalate, polyethylene glycols, polymers of acrylic and methacrylic acid and esters thereof, and mixtures of above said polymers.

Exemplary plasticizers suitable for the present invention may include plasticizers that lower the temperature of the second-order phase transition of the wall or the elastic modulus thereof, and also increase the workability of the wall and its flexibility. Plasticizers may increase or decrease the permeability of the wall to fluids including water and aqueous solutions. Plasticizers suitable for the present invention include both cyclic and acyclic plasticizers. Typical plasticizers are those selected from the group consisting of phthalates, phosphates, citrates, adipates, tartrates, sebacates, succinates, glycolates, glycerolates, benzoates, myristicates, polyethylene glycols, and polypropylene glycols. Depending on the particular plasticizer, amounts ranging from 0.1 to about 60% of the plasticizer can be used based upon the total weight of the polymer. Exemplary plasticizers for the present invention include dialkyl phthalates, dicyclalkyl phthalates, diaryl phthalates, and mixed alkylaryl as represented by dimethyl phthalate, dipropyl phthalate, dibutyl phthalate, dioctyl phthalate, di-isopropyl phthalate; alkyl and aryl phosphates such as triethyl phosphate and tributyl phosphate; alkyl citrate and citrate esters such as tributyl citrate, triethyl citrate, acetyl tributyl citrate, and acetyl triethyl citrate; alkyl adipates such as dioctyl adipate and diethyl adipate; dialkyl tartrates such as diethyl tartrate and dibutyl tartrate; sebacates such as diethyl sebacate, dibutyl sebacate, and dipropyl sebacate. Other plasticizers include polyethylene glycols, camphor, liquid sorbitol, triacetin, castor oil, olive oil, sesame oil, substituted epoxides, and mixtures of above.

It is generally desirable from a preparation standpoint to mix the polymer in a solvent. Exemplary solvents suitable for manufacturing the wall of the instant delivery system may include inorganic and organic solvents that do not adversely harm the core, wall, and the materials forming the final wall. The solvents broadly include members selected from the group consisting of aqueous solvents, alcohols, ketones, esters, ethers, aliphatic hydrocarbons, halogenated solvents, cycloaliphatic, aromatic, heterocyclic solvents and mixtures thereof. Water based latex forms of the suitable polymers also fall within the scope of the present invention. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl ethyl ketone, methyl propyl ketone, n-hexane, ethyl lactate, n-heptane, ethylene glycol monoethyl acetate, methylene dichloride, ethylene dichloride, propylene dichloride, carbon tetrachloride, nitroethane, nitropropane, tetrachloroethane, ethyl ether, isopropyl ether, cyclohexane, cyclooctane, dimethylbromamide, benzene, toluene, water, and mixtures thereof such as acetone and water, acetone and methanol, methylene dichloride and methanol.

The pharmaceutical composition, in the present invention, is manufactured by standard techniques of tableting and coating. For example, in one of the procedures the therapeutically active ingredient, alkalinizing agent(s)/buffer(s), and osmagents are dry blended. The solubility-modifying agent, in the present invention, is alkaline in nature or it can be buffer, and is in immediate contact with the therapeutically active ingredient and is able to alter the micro environmental pH and thus, the solubility of the therapeutically active ingredient. These components are then mixed with conventional excipients so as to form core tablet. Optionally, the core composition can be prepared by the techniques of slugging or wet granulation. In wet granulation, the drug is blended with the other excipients including allcalinizing agent(s)/buffer(s) and osmagent using water, alcohol, or an organic co solvent such as isopropyl alcohol/methylene chloride, 80/20, V/V as the granulation fluid. The wet granules are dried and passed through a 20 or 22-mesh screen and blended with lubricant and glidant in a mixer. The blend is then compressed in the form of a tablet. Optionally, the drug and the solubility modifier can be dissolved in a common aqueous or organic solvent and after evaporation to dryness, the residue can be mixed with osmagent and other excipients needed to form the core tablet. The compressed tablet is then coated with a membrane wall. The wall forming composition can be applied using the techniques of press coating, spraying, dipping, or air suspension techniques. The wall surrounding the tablet core is prepared by using the mixture of water insoluble semi-permeable membrane forming polymer and water-soluble polymer and it is possible to control the permeability of the membrane by varying the ratio of semi-permeable and permeable membrane forming polymers. In general, increasing the concentration of water-insoluble semi-permeable membrane forming polymer will decrease the membrane permeability and the drug release. On the other hand, increasing the concentration of water-soluble polymer will increase the membrane permeability and the drug release. In operation, the core compartment imbibes aqueous fluids from the surrounding environment across the rate controlling membrane. Dissolution of the alkalinizing agent(s)/buffer(s), which are in immediate contact with the therapeutically active ingredient, results in the elevation of the micro environmental pH of the core above the pKa of the therapeutically active ingredient. The solubility of the therapeutically active ingredient and, thus, its release from the pharmaceutical composition is improved by the change in the micro environmental pH. By adjusting the amount and/or type of alkalinizing agent(s)/buffer(s), based upon their ability to modulate the micro environmental pH, the release profile of the drug can be adjusted to meet the desired kinetic profile.

The novelty in the present invention is that a novel pharmaceutical composition, consisting of a tablet core of a therapeutically active ingredient that is weakly acidic in nature, solubility modifier, in immediate contact with the therapeutically active ingredient, and osmagent, is prepared and coated with a membrane wall forming composition consisting of a semi-permeable membrane forming polymer, permeable membrane forming polymer, and plasticizer(s). Thus, the pharmaceutical composition, in the present invention, is manufactured using less number of steps as compared to the prior art and yet effective in extended release of a therapeutically active ingredient having limited solubility in the aqueous and biological fluids.

### EXAMPLES

The following examples are given by way of illustration and therefore should not be construed to limit the scope of the present invention. Examples 1, 5, 7, and 8 illustrate the invention. Examples 2-4 and 6 are for comparative purpose only.

In the examples 1-6, glipizide, an oral sulfonylurea drug prescribed for the treatment of non-insulin dependent diabetes mellitus (NIDDM) is used as a model drug. Glipizide is a weakly acidic drug having a pKa of 5.9 and is practically insoluble in water. The limited solubility of glipizide would preclude its incorporation into conventional osmotic pharmaceutical composition. In the following examples, poor aqueous solubility of glipizide is improved by incorporation of alkalinizing agent tris(hydroxymethyl)aminomethane (commonly named as TRIS buffer). This permits the successful formulation of a weakly acidic drug having limited solubility in the aqueous and biological fluids.

### Example 1

A pharmaceutical composition for extended release of a weakly acidic drug, glipizide, is manufactured as follows

Core tablets of glipizide were prepared as follows

| **S. No.** | **Ingredients** | **% w/w** | **Grams** | **mg/tablet** |
|---|---|---|---|---|
| 1 | Glipizide | 2.78 | 6.95 | 10.00 |
| 2 | TRIS buffer | 48.61 | 121.53 | 175.00 |
| 3 | Mannitol | 29.89 | 74.73 | 107.60 |
| 4 | Sodium chloride | 9.72 | 24.30 | 35.00 |
| 5 | Polyvinyl pyrrolidone | 5.00 | 12.50 | 18.00 |
| 6 | Magnesium stearate | 1.50 | 3.75 | 5.40 |
| 7 | Talc | 2.00 | 5.00 | 7.20 |
| 8 | Aerosil | 0.50 | 1.25 | 1.80 |

TRIS buffer (Loba Chemie, India) was mixed with directly compressible mannitol (Pearlitol SD 200, Roquette, France) and sodium chloride (Loba Chemie, India) and then passed through a 30-mesh sieve (British Standard Sieves, BSS). Glipizide was mixed with a part of the portion obtained above and after mixing, was passed through a 30-mesh sieve (BSS). The blend was mixed for 10 minutes and polyvinyl pyrrolidone (Plasdone K 29/32, ISP, USA) was added to the mixture. The mixture was granulated with ethanol and the resulting wet dough was passed through 18-mesh sieve (BSS). The wet granules so obtained were dried at 50°C for 10 minutes and the dry granules were passed through 22-mesh sieve (BSS) to break the agglomerates. These sized granules were then blended with magnesium stearate, talc, and aerosil (all 60-mesh passed) and compressed in the form of biconvex tablets having an average weight of 360 mg using a single stroke tablet-punching machine (Cadmach CMS-25, India) fitted with 10.00 mm round standard concave punches. Around 500 of these tablets were placed in a laboratory scale perforated coater (Ganscoater-GAC 250, Gansons, India) along with 200 grams of filler tablets (tablets made using 7.00 mm round deep concave punches and containing microcrystalline cellulose, starch, dibasic calcium phosphate, magnesium stearate, and aerosil) and coated with a coating solution comprising of

| **S. No.** | **Ingredients** | **% w/w** | **Grams** |
|---|---|---|---|
| 1 | Cellulose acetate | 2.58 | 55.00 |
| 2 | Triacetin | 0.26 | 5.50 |
| 3 | PEG-400 | 0.52 | 11.00 |
| 4 | Polyvinyl pyrrolidone | 0.64 | 13.75 |
| 5 | Methanol | 24.00 | 511.63 |
| 6 | Methylene chloride | 72.00 | 1534.88 |

The coating solution was prepared by adding cellulose acetate having a molecular weight of approximately 37, 000 and acetyl value of 40% (Fluka, Switzerland) to the mixture of methylene chloride and methanol. After the entire polymer was dissolved, polyvinyl pyrrolidone (Plasdone K 29/32, ISP, USA) was added with continuous stirring. Finally, triacetin (Acros Organics, USA) and PEG-400 (S.D. Fine-Chem Ltd., India) were added and thoroughly mixed to give the final coating solution. The coating solution in all the examples contained the total solid content of approximately 4% w/w. The filler and active tablets were placed in the coating pan and the heated air was passed through the tablet bed.

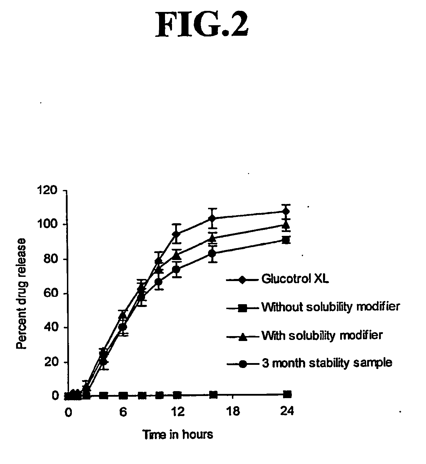

The pan was rotated at 18-20 rpm. When the outlet air temperature reached 28°C, the coating solution was applied through the atomizing nozzle at the rate of 7-8 ml/minute with atomization at 1 Kg/Cm². Sufficient coating solution was applied until a % weight increase of 12.65% was achieved on the active tablets. The active tablets were dried in an oven for 16 hours at 50°C. The release studies of these tablets and marketed extended release formulations of glipizide (Glucotrol XL 10 mg, Pfizer Inc., USA) was conducted in 1000 ml of simulated intestinal fluid, pH 6.8, without enzymes using USP type 1 (basket) apparatus at 100 rpm. The plot of percent drug released versus time is shown in figure 2. The release profile of the composition as prepared above was compared to that of the marketed extended release formulation (Glucotrol XL) using a similarity factor, *f*₂, as mentioned in Pharmaceutical Technology, vol. 20, pp 64-74. This similarity factor has been adopted by the Center for Drug Evaluation and Research (Food and Drug Administration, USA) as a criterion for the assessment of the similarity between two in vitro dissolution profiles. In order to consider the two dissolution profiles to be similar, the *f*₂ value should be between 50 and 100. The in vitro release data of the composition manufactured as above sand that of marketed extended release formulation was analyzed and *f*₂ value was calculated, which was found to be 59. Thus, it can be concluded that both the dissolution profiles are similar with respect to each other.

The pharmaceutical composition as prepared above was packed in 0.04 mm thick aluminum foil laminated with PVC and stored in stability chambers maintained at 40°C and 75% RH for 3 months. The release studies of tablets after 3 months of storage at the above condition was conducted in 1000 ml of simulated intestinal fluid, pH 6.8, without enzymes using USP type 1 (basket) apparatus at 100 rpm and the release profile obtained is shown in figure 2.

To study the effect of solubility modifier (TRIS buffer), tablet compositions of glipizide were prepared (without TRIS buffer, which was substituted with an equal quantity of mannitol) and coated as per the procedure outlined above. Sufficient coating solution was applied until a % weight increase of 13.18% was achieved on the active tablets. The active tablets were dried in an oven for 16 hours at 50°C. The release study of these tablets was conducted in 1000 ml of simulated intestinal fluid, pH 6.8, without enzymes using USP type 1 (basket) apparatus at 100 rpm. The plot of percent drug released versus time is shown in figure 2.

The results shown in figure 2 depicts that there was no drug release from the pharmaceutical composition that did not contain the solubility modifier. On the other hand, the pharmaceutical compositions containing a solubility modifier and prepared in accordance with the present invention exhibits extended release of a weakly acidic drug having a limited solubility in the aqueous fluids. It has been shown in the preceding example that it is possible to improve the solubility of a weakly acidic drug through the use of alkalinizing agent. It has also been shown that meaningful release rates can be obtained for a drug having limited solubility in the aqueous fluids through proper choice of alkalinizing agent. Moreover, the formulations can be expected to have a reasonable shelf life as shown by the accelerated stability data for 3 months, which demonstrates that the release profile is similar to that of initial samples.

### Example 2

Core tablets of glipizide of following composition were prepared as per the procedure outlined in example 1

| **S. No.** | **Ingredients** | **% w/w** | **Grams** | **mg/tablet** |
|---|---|---|---|---|
| 1 | Glipizide | 2.78 | 2.78 | 10.00 |
| 2 | TRIS buffer | 48.61 | 48.61 | 175.00 |
| 3 | Mannitol | 30.39 | 30.39 | 109.40 |
| 4 | Sodium chloride | 9.72 | 9.72 | 35.00 |
| 5 | Polyvinyl pyrrolidone | 5.00 | 5.00 | 18.00 |
| 6 | Magnesium stearate | 1.00 | 1.00 | 3.60 |
| 7 | Talc | 2.00 | 2.00 | 7.20 |
| 8 | Aerosil | 0.50 | 0.50 | 1.80 |

100 of these tablets were placed in a laboratory scale 10" perforated coater along with 350 grams of filler tablets (tablets made using 7.00 mm round deep concave punches and containing microcrystalline cellulose, starch, dibasic calcium phosphate, magnesium stearate, and aerosil) and coated with a coating solution comprising of:

| **S. No.** | **Ingredients** | **% w/w** | **Grams** |
|---|---|---|---|
| 1 | Cellulose acetate | 2.58 | 65.00 |
| 2 | Triacetin | 0.26 | 6.50 |
| 3 | PEG-400 | 0.52 | 13.00 |
| 4 | Polyvinyl pyrrolidone | 0.64 | 16.25 |
| 5 | Methanol | 24.00 | 604.65 |
| 6 | Methylene chloride | 72.00 | 1813.95 |

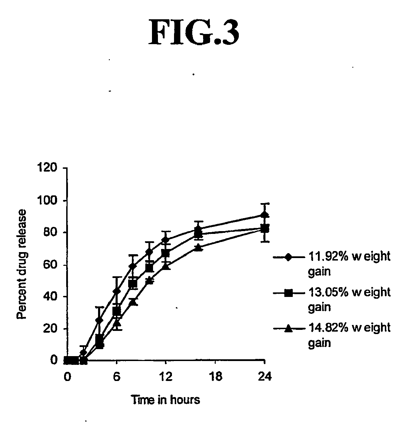

The coating solution was prepared as per the procedure outlined in example 1. The filler and active tablets were placed in the coating pan and the heated air was passed through the tablet bed. The pan was rotated at 18-20 rpm. When the outlet air temperature reached 28°C, the coating solution was applied through the atomizing nozzle at the rate of 7-8 ml/minute with atomization at 1 Kg/Cm². Sufficient coating solution was applied until a % weight increase of 11.92% was achieved on the active tablets. 30 of the active tablets were withdrawn and coating continued until % weight increase of 13.05% on active tablets was achieved. 30 of the tablets were removed and coating continued to achieve a weight gain of 14.82% on the active tablets. The active tablets were dried in an oven for 16 hours at 50°C. The release study of these tablets was conducted in 1000 ml of simulated intestinal fluid, pH 6.8, without enzymes using USP type 1 (basket) apparatus at 100 rpm. The plot of percent drug released versus time is shown in figure 3.

The results shown in figure 3 depicts that the pharmaceutical compositions prepared in accordance with the present invention exhibits extended release of glipizide for prolonged period of time. In this example cellulose acetate is a semi-permeable membrane-forming polymer and polyvinyl pyrrolidone is a permeable membrane-forming polymer. It is evident from the figure that the drug release is affected by the percentage weight gain of polymer solution on the active tablets and it is possible to modulate the release as per the requirements by varying this membrane parameter.

### Example 3

Example 2 was repeated except that the following coating compositions were used to explore the possibility of varying the concentrations of a water-soluble plasticizer (PEG-400) and plasticizer having limited solubility in water (Triacetin).

| **S. No.** | **Ingredients** | **---------------------% w/w-----------------------** | | | |
|---|---|---|---|---|---|
| | | **A** | **B** | **C** | **D** |
| 1 | Cellulose acetate | 2.580 | 2.420 | 2.420 | 2.760 |
| 2 | Triacetin | 0.258 | 0.242 | 0.484 | 0.552 |
| 3 | PEG-400 | 0.516 | 0.726 | 0.484 | - |
| 4 | Polyvinyl pyrrolidone | 0.645 | 0.605 | 0.605 | 0.690 |
| 5 | Methanol | 24.00 | 24.00 | 24.00 | 24.00 |
| 6 | Methylene chloride | 72.00 | 72.00 | 72.00 | 72.00 |

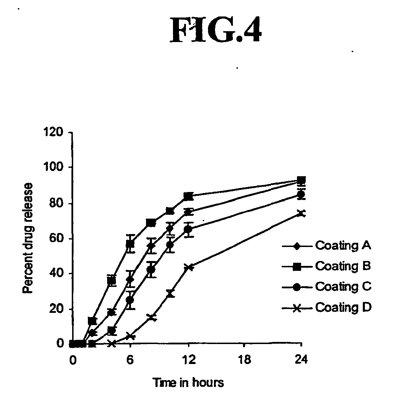

The coating solution was prepared as per the procedure outlined in example 1 and tablets were coated as described in example 1. The coating was continued until a weight gain of approximately 12.75% was achieved on the active tablets. The active tablets were dried in an oven for 16 hours at 50°C. The release profile of glipizide from the active tablets is shown in figure 4. It is evident from the figure that increasing the concentration of a relatively water-soluble plasticizer like PEG-400 increases drug release. On the other hand, increasing the concentration of a relatively water-insoluble plasticizer like triacetin or decreasing the concentration of a relatively water-soluble plasticizer like PEG-400 decreases the drug release. PEG-400 is completely miscible in water whereas, 1 part of triacetin is soluble in 14 parts of water at 20°C. Hence, it is possible to modulate the drug release as per the requirements by proper choice and varying the concentrations of plasticizers.

### Example 4

Example 2 was repeated except that the following coating compositions were used to study the effect of level of water-soluble permeable membrane forming polymer (PVP) on drug release.

| **S. No.** | **Ingredients** | **------------------% w/w----------------** | |
|---|---|---|---|
| | | **E** | **F** |
| 1 | Cellulose acetate | 3.08 | 2.22 |
| 2 | Triacetin | 0.31 | 0.22 |
| 3 | PEG-400 | 0.62 | 0.44 |
| 4 | Polyvinyl pyrrolidone (PVP) | - | 1.11 |
| 5 | Methanol | 24.00 | 24.00 |
| 6 | Methylene chloride | 72.00 | 72.00 |

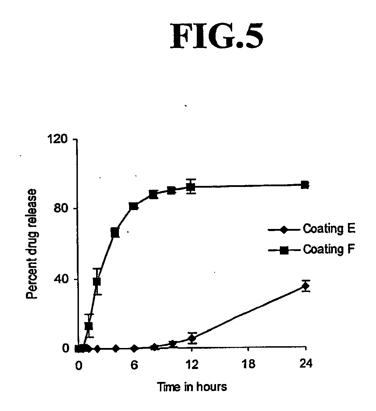

The coating solution was prepared as per the procedure outlined in example 1 and tablets were coated as described in example 1. The coating was continued until a weight gain of approximately 12.50% was achieved on the active tablets. The active tablets were dried in an oven for 16 hours at 50°C. The release profile of glipizide from the active tablets is shown in figure 5. It is clearly evident from the figure that the drug release increases as the level of permeable membrane forming polymer increases and thus, it is possible to control the release profile of drugs by varying the level of permeable membrane forming polymer.

### Example 5

Example 2 was repeated except that the following coating composition (Coating composition G) was used to explore the possibility of using ethylcellulose as a semi-permeable membrane forming polymer

| **S. No.** | **Ingredients** | **% w/w** | **Grams** |
|---|---|---|---|
| 1 | Ethyl cellulose | 2.74 | 40.00 |
| 2 | Polyvinyl pyrrolidone | 1.52 | 22.19 |
| 3 | Propylene glycol | 0.73 | 10.66 |
| 4 | Methylene chloride | 57.00 | 832.12 |
| 5 | Ethanol | 38.00 | 554.74 |

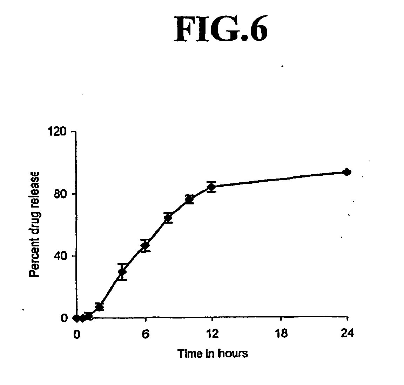

The coating solution was prepared by adding ethyl cellulose (Ethocel standard 10 cp premium, Colorcon Asia Pvt. Lt., Mumbai) to the mixture of methylene chloride and ethanol. After the entire polymer was dissolved, PVP was added with continuous stirring. Finally, propylene glycol was added and thoroughly mixed to give the final coating solution. The tablets were coated as per the conditions outlined in example 1 until a final weight gain of 12.09% was achieved on the active tablets. The active tablets were dried in an oven for 16 hours at 50°C. The release profile of glipizide from the active tablets is shown in figure 6 and it is clearly evident from the figure that the drug release is controlled for a prolonged period.

### Example 6

### Direct Compression

Core tablets of glipizide as per the following formula

| **S. No.** | **Ingredients** | **% w/w** | **Grams** | **mg/tablet** |
|---|---|---|---|---|
| 1 | Glipizide | 2.67 | 1.50 | 10.00 |
| 2 | TRIS buffer | 56.33 | 31.69 | 211.25 |
| 3 | Mannitol | 20.49 | 11.53 | 76.85 |
| 4 | Sodium chloride | 16.00 | 9.00 | 60.00 |
| 5 | Magnesium stearate | 4.00 | 2.25 | 15.00 |
| 6 | Aerosil | 0.51 | 0.29 | 1.90 |

TRIS buffer was mixed with directly compressible mannitol and sodium chloride and passed through a 30-mesh sieve (BSS). Glipizide was mixed with a part of the portion obtained above and after mixing, was passed through a 30-mesh sieve (BSS). The blend, after mixing for 10 minutes, was mixed with magnesium stearate and aerosil (both 60-mesh passed) and compressed in the form of biconvex tablets having an average weight of 375 mg using a single stroke tablet-punching machine fitted with 10.00 mm round standard concave punches. The tablets were coated as per the procedure outlined in example 1 except that the following coating compositions were used to explore the possibility of using hydroxypropylmethyl cellulose (HPMC) as the permeable membrane forming polymer.

| **S. No.** | **Ingredients** | **---------------------% w/w-----------------------** | | |
|---|---|---|---|---|
| | | **H** | **I** | **J** |
| 1 | Cellulose acetate | 2.420 | 2.290 | 2.350 |
| 2 | Triacetin | 0.484 | 0.458 | 0.470 |
| 3 | PEG-400 | 0.484 | 0.687 | - |
| 4 | HPMC | 0.605 | 0.572 | 0.587 |
| 5 | Polyvinyl pyrrolidone | - | - | 0.587 |
| 6 | Methanol | 24.000 | 24.000 | 24.000 |
| 7 | Methylene chloride | 72.000 | 72.000 | 72.000 |

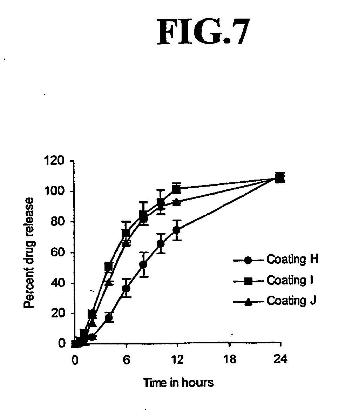

The coating solution was prepared by adding cellulose acetate having a molecular weight of approximately 37, 000 and acetyl value of 40% to the mixture of methylene chloride and methanol. After the entire polymer was dissolved, HPMC (Pharmacoat 606, Shin-Etsu, Japan) was added with continuous stirring. Finally, triacetin and PEG-400 (or Polyvinyl pyrrolidone in case of coating composition J) were added and thoroughly mixed to give the final coating solution. The coating was applied as per the procedure outlined in example 1. The coating was continued until a weight gain of approximately 12.00% was achieved on the active tablets. The active tablets were dried in an oven for 16 hours at 50°C. The release profile of glipizide from the active tablets is shown in figure 7. It is clearly evident from the figure that the release of glipizide is controlled and HPMC can also be utilized as a permeable membrane forming polymer.

Following table shows the effect of variation of critical formulation variables in the selected compositions manufactured in the above examples on percent drug release after 6, 12, and 24 hours of dissolution testing.

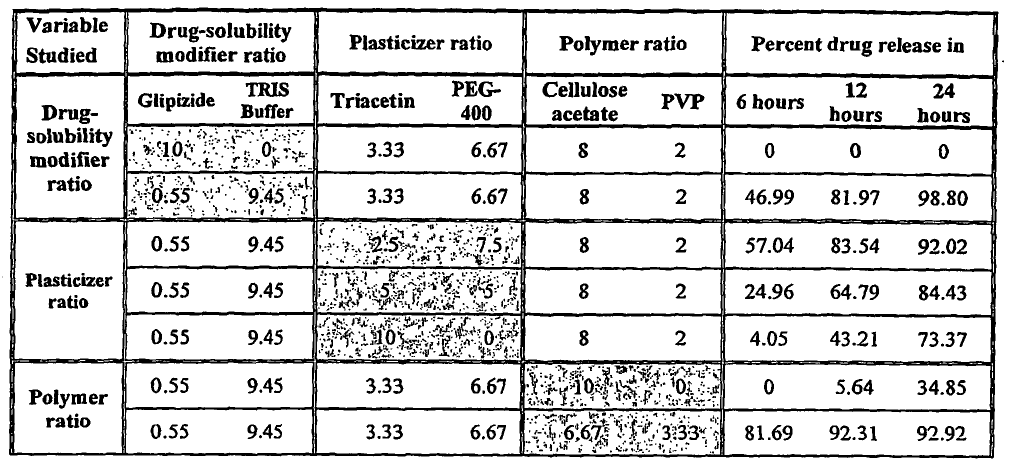

It is evident from the table that presence of solubility modifier (alkalinizing agent or a buffer) is necessary in the pharmaceutical composition so that the drug release may take place. Increase in the concentration of a water-soluble plasticizer (PEG-400) increase the drug release at each time point and maximal extent of drug release after 24 hours. On the other hand, increase in the concentration of plasticizer with limited water solubility (Triacetin) decreases drug release. It is also evident from the table that, increase in the concentration of water-soluble polymer (PVP) in the membrane increases the drug release. Thus, in the preceding examples, it was shown that it is possible to control the release of the therapeutically active ingredient by modulating the critical formulation variables.

### Example 7

In the following example, aspirin, which is used as an antipyretic, analgesic, and anti-inflammatory agent and at low dose is effective as an anti-platelet agent, is used as a model drug. Aspirin is a weakly acidic drug having a pKa of 3.5 and it is slightly soluble in water. Core tablets of aspirin were prepared as follows:

| **S. No.** | **Ingredients** | **------------------------------% w/w-------------------------------** | | |
|---|---|---|---|---|
| | | **Core I** | **Core II** | **Core III** |
| 1 | Aspirin | 30.00 | 30.00 | 30.00 |
| 2 | TRIS buffer | - | 24.50 | 40.00 |
| 3 | Lactose | 64.50 | 36.50 | 21.00 |
| 4 | Polyvinyl pyrrolidone | 4.00 | 5.00 | 5.00 |
| 5 | Magnesium stearate | 1.00 | 2.00 | 2.00 |
| 6 | Talc | - | 1.50 | 1.50 |
| 7 | Aerosil | 0.50 | 0.50 | 0.50 |

Aspirin, spray dried lactose (Flowlac-100, Meggle, Germany), and TRIS buffer (except core I) were mixed and passed through a 30-mesh sieve (BSS). The blend was mixed for 10 minutes and polyvinyl pyrrolidone was added to the mixture. The mixture was granulated with ethanol and the resulting wet dough was passed through 20-mesh sieve (BSS). The wet granules so obtained were dried at 50°C for 10 minutes and the dry granules were passed through 20-mesh sieve (BSS) to break the agglomerates. These sized granules were then blended with magnesium stearate, talc (except core I), and aerosil (all 60-mesh passed) and compressed in the form of biconvex tablets using a single stroke tablet-punching machine fitted with 8.5 mm round standard concave punches. Each tablet had an average weight of 250 mg and contained 75 mg of aspirin. The tablets were coated as per the procedure outlined in example 1 using the following coating composition

| **S. No.** | **Ingredients** | **% w/w** | **Grams** |
|---|---|---|---|
| 1 | Cellulose acetate | 2.58 | 55.00 |
| 2 | Triacetin | 0.26 | 5.50 |
| 3 | PEG-400 | 0.52 | 11.00 |
| 4 | Polyvinyl pyrrolidone | 0.64 | 13.75 |
| 5 | Methanol | 24.00 | 511.63 |
| 6 | Methylene chloride | 72.00 | 1534.88 |

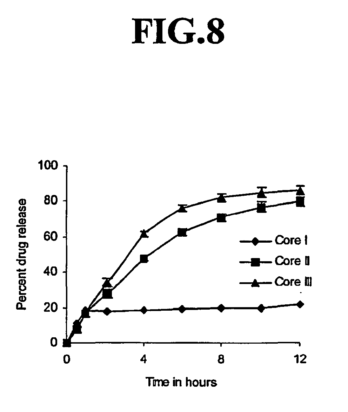

The coating was continued until a weight gain of approximately 11.00% was achieved on the active tablets. The active tablets were dried in an oven for 16 hours at 50°C. The release study of these tablets was conducted in 900 ml of acetate buffer, pH 4.5 using USP type 1 (basket) apparatus at 100 rpm. The plot of percent drug released versus time is shown in figure 8.

The results shown in figure 8 depicts that the drug release from the pharmaceutical composition that did not contain the solubility modifier was very less. On the other hand, the pharmaceutical compositions containing a solubility modifier and prepared in accordance with the present invention exhibits extended release of a weakly acidic drug having a limited solubility in the aqueous fluids. It has been shown in the preceding example that it is possible to improve the solubility of a weakly acidic drug and hence, its release by increasing the concentration of the solubility modifier. It has also been shown that meaningful release rates can be obtained for a drug having limited solubility in the aqueous fluids through proper choice of alkalinizing agent.

Following table shows the effect of variation of concentration of solubility modifier (TRIS buffer) in the compositions manufactured in the above example on percent drug release after 2, 6, and 12 hours of dissolution testing.

| **Drug-solubility modifier ratio** | | **Percent drug release in** | | |
|---|---|---|---|---|
| **Aspirin** | **TRIS Buffer** | **2 hours** | **6 hours** | **12 hours** |
| 10 | 0 | 17.7 | 19.16 | 21.76 |
| 5.5 | 4.5 | 27.68 | 62.22 | 79.69 |
| 4.29 | 5.71 | 34.07 | 75.81 | 85.63 |

It is evident from the table that the release of the therapeutically active ingredient is dependent on the concentration of solubility modifier (TRIS buffer). Increase in the concentration of TRIS buffer increase the drug release at each time point and maximal extent of drug release after 12 hours.

### Example 8

In the following example, gliclazide, an oral sulfonylurea drug prescribed for the treatment of non-insulin dependent diabetes mellitus (NIDDM) is used as a model drug. Gliclazide is a weakly acidic drug having a pKa of 5.98 and it is practically insoluble in water.

Core tablets of gliclazide were prepared as follows

| **S. No.** | **Ingredients** | **------------------------------% w/w------------------------------** | | |
|---|---|---|---|---|
| | | **Core IV** | **Core V** | **Core VI** |
| 1 | Gliclazide | 13.33 | 13.33 | 13.33 |
| 2 | TRIS buffer | - | 23.19 | 38.64 |
| 3 | Mannitol | 77.29 | 54.10 | 38.65 |
| 4 | Polyvinyl pyrrolidone | 4.00 | 4.00 | 4.00 |
| 5 | Magnesium stearate | 5.00 | 5.00 | 5.00 |
| 6 | Aerosil | 0.38 | 0.38 | 0.38 |

TRIS buffer (except core IV) and directly compressible mannitol were mixed and passed through a 30-mesh sieve (BSS). Gliclazide was mixed with a part of the portion obtained above and after mixing, was passed through a 30-mesh sieve (BSS). The blend was mixed for 10 minutes and polyvinyl pyrrolidone was added to the mixture. The mixture was granulated with water and the resulting wet dough was passed through 18-mesh sieve (BSS). The wet granules so obtained were dried at 50°C for 10 minutes and the dry granules were passed through 22-mesh sieve (BSS) to break the agglomerates. These sized granules were then blended with magnesium stearate and aerosil (60-mesh passed) and compressed in the form of biconvex tablets using a single stroke tablet-punching machine fitted with 9.5 mm round deep concave punches. Each tablet had an average weight of 300 mg and contained 40 mg of gliclazide. The tablets were coated as per the procedure outlined in example 1 using the following coating composition.

| **S. No.** | **Ingredients** | **% w/w** | **Grams** |
|---|---|---|---|
| 1 | Cellulose acetate | 2.35 | 40.00 |
| 2 | Triacetin | 0.47 | 8.00 |
| 4 | Polyvinyl pyrrolidone | 0.59 | 10.00 |
| 5 | HPMC | 0.59 | 10.00 |
| 6 | Methanol | 24.00 | 408.51 |
| 7 | Methylene chloride | 72.00 | 1225.53 |

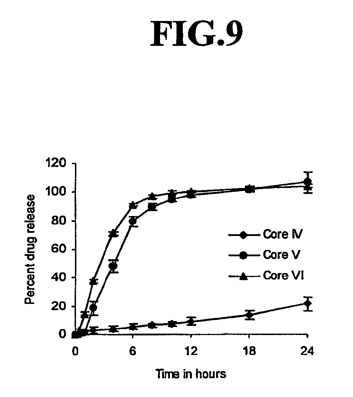

The coating was applied as per the procedure outlined in example 1. The coating was continued until a weight gain of approximately 12.00% was achieved on the active tablets. The active tablets were dried in an oven for 16 hours at 50°C. The release study of these tablets was conducted in 1000 ml of phosphate buffer, pH 7.4 using USP type 1 (basket) apparatus at 100 rpm. The plot of percent drug released versus time is shown in figure 9.

The results shown in figure 9 depicts that the drug release from the pharmaceutical composition that did not contain the solubility modifier was very less. On the other hand, the pharmaceutical compositions containing a solubility modifier and prepared in accordance with the present invention exhibits extended release of a weakly acidic drug having a limited solubility in the aqueous fluids. It has been shown in the preceding example that it is possible to improve the solubility of a weakly acidic drug and hence, its release by increasing the concentration of the solubility modifier. It has also been shown that meaningful release rates can be obtained for a drug having limited solubility in the aqueous fluids through proper choice of alkalinizing agent.

Following table shows the effect of variation of concentration of solubility modifier (TRIS buffer) in the compositions manufactured in the above example on percent drug release after 6, 12, and 24 hours of dissolution testing.

| **Drug-solubility modifier ratio** | | **Percent drug release in** | | |
|---|---|---|---|---|
| **Gliclazide** | **TRIS Buffer** | **6 hours** | **12 hours** | **24 hours** |
| 10 | 0 | 5.62 | 9.35 | 21.68 |
| 3.65 | 6.35 | 79.49 | 97.92 | 106.95 |
| 2.56 | 7.44 | 91.02 | 100.19 | 104.37 |

It is evident from the table that the release of the therapeutically active ingredient is dependent on the concentration of solubility modifier (TRIS buffer). Increase in the concentration of TRIS buffer increase the drug release at each time point and maximal extent of drug release after 24 hours.

Thus, a novel pharmaceutical composition has been developed for a therapeutically active ingredient that is weakly acidic in nature and having a limited solubility in the aqueous and biological fluids, through selection of alkalinizing agents that are in immediate contact with the therapeutically active ingredient and, which after coming in contact with the aqueous fluids, elevate the micro environmental pH of the core above the pKa of the therapeutically active ingredient, and also the rate controlling membrane wall applied to the core is much simpler in nature comprising of semi-permeable and permeable polymers, and do not necessitate creation of delivery orifice through an additional step.

### The main advantages of the present invention are:

1. The pharmaceutical composition can be used for extended delivery of a therapeutically active ingredient, limited solubility of which would preclude its incorporation into conventional osmotic compositions.
2. The pharmaceutical composition is simple to manufacture and the solubility of the therapeutically active ingredient can be easily improved by proper choice of alkalinizing agent(s)/buffer(s), based upon their ability to modulate the micro environmental pH.
3. The pharmaceutical composition does not require sophisticated techniques like laser drilling across the membrane wall to form passageway(s) for the release of drug, and proper choice of semi-permeable and permeable polymers can be made to control the release of the drug.
4. The pharmaceutical composition requires minimum number of manufacturing steps and is simple in design and easily amenable to mass production.

## Claims

1. A pharmaceutical composition for sustained release of a therapeutically active moiety, said composition comprising a tablet core surrounded by a release rate controlling membrane, said tablet core consisting of
(i) the therapeutically active moiety having limited solubility in the aqueous fluids, weakly acidic in nature and having a pKa between 2.5 to 7.5,
(ii) an alkalinizing agent or a buffer compound in immediate contact with the above said therapeutically active moiety,
(iii) an osmotically effective solute that is soluble in water and capable of exhibiting an osmotic pressure gradient across the release rate controlling membrane against the external fluids, and
(iv) optionally containing one or more pharmaceutically acceptable excipients and polymers,
said release rate controlling membrane consisting of:
(i) a semi-permeable membrane forming polymer which is insoluble in water but partially permeable to aqueous fluids and substantially impermeable to the core composition,
(ii) a permeable membrane forming polymer which is soluble in water and permeable to aqueous fluids, and to at least one of the moiety of the core composition; and
(iii) at least one plasticizer ranging between 2 to 60 % by weight, based on the - total weight of dry polymers.

2. The composition according to claim 1, wherein semi-permeable membrane forming polymer and permeable membrane forming polymer together with plasticizer are coated on the tablet core and dried to get the release rate controlling polymer membrane.

3. A composition according to claim 1, wherein the therapeutically active moiety is a drug that act on peripheral nerves, adrenergic receptors, cholinergic receptors, nervous system, skeletal muscles, cardiovascular, smooth muscles, blood circulatory system, synaptic sites, neuroeffector junctional sites, endocrine and hormone system, immunological system, reproductive system, skeletal system, autocoid systems, alimentary and excretory systems, inhibitory or autocoids and histamine systems, and those materials that act on the central nervous system such as hypnotics and sedatives.

4. A composition according to claim 1, wherein the therapeutically active moiety is selected from a group consisting of acetazolamide, acetyl salicylic acid, p-amino salicylic acid, captropil, carbenicillin, carbenoxolone, chlorpropamide, clofibrate, diclofenac, diflunisal, ethacrynic acid, etodolac, fenoprofen, furosemide, gliclazide, glimepiride, glipizide, glyburide, ibuprofen, indomethacin, ketoprofen, naproxen, nimesulide, tolazamide, tolbutamide, tolmentin and zomepirac.

5. A composition according to claim 1, wherein the therapeutically active moiety is preferably selected from hypoglycemic agent and anti-inflammatory agent.

6. A composition according to claim 5, wherein the hypoglycemic agent used is selected from the category of sulfonylurea.

7. A composition according to claim 6, wherein the sulfonylurea used is selected from the group consisting of glipizide, gliclazide, glimepiride, and glyburide.

8. A composition according to claim 5, wherein the anti-inflammatory agent is selected from the group consisting of aspirin, paracetamol, ibuprofen, indomethacin, ketoprofen, naproxen, nimesulide, tolmentin and zomepirac preferably selected from Asprin.

9. A composition according to claim 1, wherein the dose of the therapeutically active moiety per tablet ranges between 0.1 mg to 600 mg.

10. A composition according to claim 1, wherein the alkalinizing agent or the buffer used is soluble in water and improves the solubility of therapeutically active moiety in the aqueous fluids by increasing the micro environmental pH of the core above the pKa value of the therapeutically active moiety.

11. A composition according to claim 10, wherein the alkalinizing agent used is selected from the group consisting of sodium bicarbonate, potassium bicarbonate, sodium citrate, potassium citrate, tris(hydroxymethyl)aminomethane, meglumine, and/or the mixture thereof.

12. A composition according to claim 10, wherein the buffer used is selected from the group consisting of sodium phosphates, potassium phosphates, sodium citrate, potassium citrate, sodium acetate, tris(hydroxymethyl)aminomethane, and/or mixtures thereof.

13. A composition according to claim 1, wherein the ratio of the therapeutically active ingredient to the alkalinizing agent is in the range of 0.1:9.9 to 7:3.

14. A composition according to claim 1, wherein the ratio of the therapeutically active ingredient to the buffer is in the range of 0.1:9.9 to 7:3.

15. A composition according to claim 1, wherein the osmotically effective solute used is selected from the group consisting of sodium chloride, potassium chloride, mannitol, sorbitol, and carbohydrates selected from the group consisting of sucrose, glucose, fructose, dextrose, lactose, and mixtures of above said osmagents.

16. A composition according to claim 15, wherein the osmotically effective solute used is preferably selected from the group consisting of sodium chloride, mannitol, and lactose.

17. A composition according to claim 1, wherein the core components exerts osmotic gradient across the wall of release rate controlling membrane against the external fluids.

18. A composition according to claim 1, wherein the semi permeable membrane forming polymer is water insoluble, which allows water to permeate and substantially prevents permeability of compositions of the tablet core.

19. A composition according to claim 1, wherein the semi-permeable membrane forming polymer is selected from group consisting of cellulose acetate, cellulose acetate butyrate, cellulose acetate propionate, ethyl cellulose, polymers of acrylic and methacrylic acid and esters thereof.

20. A composition according to claim 19, wherein the preferred semi-permeable membrane forming polymer is selected from cellulose acetate and ethyl cellulose.

21. A composition according to claim 1, wherein the permeable membrane forming is a water-soluble, which allows water to permeate along with at least one of the components of the core.

22. A composition according to claim 1, wherein the permeable membrane forming polymer is selected from the group consisting of polyvinyl alcohol, polyvinyl pyrrolidone, cellulose ethers, polyethylene glycols, polymers of acrylic and methacrylic acid and esters thereof.

23. A composition according to claim 22, wherein the preferred permeable membrane forming polymer used is selected from polyvinyl pyrrolidone and hydroxypropylmethyl cellulose.

24. A composition according to claim 1, wherein the ratio of semi-permeable water insoluble polymer membrane to permeable water soluble polymer membrane is in the range of 9:1 to 1:9, preferably in the range of 9:1 to 3:7.

25. A composition according to claim 1, wherein the plasticizer used is having controlled solubility in water.

26. A composition according to claim 25, wherein the plasticizer used is selected from the group consisting of dibutyl sebacate, diethyl phthalate, dibutyl phthalate, triacetin, triethyl citrate, tributyl citrate, castor oil, propylene glycol, glycerol, polyethylene glycols, liquid sorbitol, and/or mixture thereof.

27. A composition according to claim 1, wherein by adjusting the polymer forming membrane weight coated on the tablet core, thickness of the membrane wall is controlled.

28. A composition according to claim 1, wherein the thickness of the membrane wall ranges between 1 to 1000 microns

29. A composition according to claim 28, wherein the preferred thickness of the membrane wall ranges between 50 to 500 microns

30. A composition according to claim 1, wherein the pharmaceutically acceptable excipient used is (are) selected from the group consisting of magnesium stearate, talc and aerosil.

31. A composition according to claim 1, from wherein the mechanism of release of the therapeutically active ingredient is based on combination of osmotic pumping and diffusion.

32. A process for the preparation of pharmaceutical composition as claimed in claim 1 for sustained release of a therapeutically active moiety, said process comprising:
a. preparing a core composition by dry blending a therapeutically active moiety, an alkalinizing agent or a buffer compound, an osmotically effective solute and optionally containing one or more pharmaceutically acceptable excipients and polymers, or
b. preparing the core composition by slugging or wet granulation techniques by blending the drug with the other excipients including alkalinizing agent(s)/buffer(s) and osmagent using water, alcohol, or an organic co solvent such as isopropyl alcohol/methylene chloride, in the ratio 80/20, V/V as the granulation fluid to obtain wet granules, or
c. by dissolving the drug and the solubility modifier in a common aqueous or organic solvent and after evaporation to dryness, mixing the residue with osmagent and other excipients needed to obtain core composition,
d. compressing the above core composition obtained in steps (a), (b) and (c) to obtain tablets core by using conventional tablet making machine,
e. preparing coating solution by dissolving required amount of semi permeable membrane forming polymer in a solvent selected from methylene chloride, methanol, ethanol or mixture thereof,
f. adding permeable membrane forming polymer and one or more plasticizer to the solution of step (e) with continuous stirring,
g. coating the compressed tablet of step (d) with the coating solution of step (f) by using the techniques selected from press coating, spraying, dipping, or air suspension techniques and
h. drying the coated tablet core obtained in the step (g) at 45-60°C for about 16 hours to obtain the composition for sustained release and packing the tablets by conventional methods.

33. The process according to claim 32 wherein in step (a), the mixture obtained by dry blending is passed through standard sieves to obtain uniform particle size so as to form core composition

34. The process according to claim 32 wherein in step (b), the wet granules are dried at a temperature ranging between 40 and 60°C for about 10 minutes, passing the granules through 20-22 standard sieves to break agglomerates and to making it uniform particle size to obtain core composition.

35. The process according to claim 32, wherein the therapeutically active moiety is selected from a group consisting of acetazolamide, acetyl salicylic acid, p-amino salicylic acid, captropil, carbenicillin, carbenoxolone, chlorpropamide, clofibrate, diclofenac, diflunisal, ethacrynic acid, etodolac, fenoprofen, furosemide, gliclazide, glimepiride, glipizide, glyburide, ibuprofen, indomethacin, ketoprofen, naproxen, nimesulide, tolazamide, tolbutamide, tolmentin and zomepirac.

36. The process according to claim 32, wherein the therapeutically active moiety is preferably selected from hypoglycemic agent and anti-inflammatory agent.

37. The process according to claim 36, wherein the hypoglycemic agent used is selected from the category of sulfonylurea.

38. The process according to claim 37, wherein the sulfonylurea used is selected from the group consisting of glipizide, gliclazide, glimepiride, and glyburide.

39. The process according to claim 36, wherein the anti-inflammatory agent is selected from group consisting of aspirin, paracetamol, ibuprofen, indomethacin, ketoprofen, naproxen, nimesulide, tolmentin and zomepirac preferably selected from Asprin.

40. The process according to claim 32, wherein the dose of the therapeutically active moiety per tablet ranges between 0.1 mg to 600 mg.

41. The process according to claim 32, wherein the alkalinizing agent used is selected from the group consisting of sodium bicarbonate, potassium bicarbonate, sodium citrate, potassium citrate, tris(hydroxymethyl)aminomethane, meglumine, and/or the mixture thereof..

42. The process according to claim 32, wherein the buffer used is selected from the group consisting of sodium phosphates, potassium phosphates, sodium citrate, potassium citrate, sodium acetate, tris(hydroxymethyl)aminomethane, and/or mixtures thereof.

43. The process according to claim 32, wherein the ratio of the therapeutically active ingredient to the alkalinizing agent is in the range of 0.1:9.9 to 7:3.

44. The process according to claim 32, wherein the ratio of the therapeutically active ingredient to the buffer is in the range of 0.1:9.9 to 7:3.

45. The process according to claim 32, wherein the osmotically effective solute used is selected from the group consisting of sodium chloride, potassium chloride, mannitol, sorbitol, and carbohydrates selected from the group consisting of sucrose, glucose, fructose, dextrose, lactose, and mixture thereof.

46. The process according to claim 45, wherein the osmotically effective solute used is preferably selected from the group consisting of sodium chloride, mannitol, and lactose.

47. The process according to claim 32, wherein the excipient(s) used is(are) selected from group consisting of magnesium stearate, talc and aerosil.

48. The process according to claim 32, wherein the polymer(s) used is(are) selected from group consisting of polyvinyl alcohol, polyvinyl pyrrolidone, cellulose ethers, polyethylene glycols, cellulose acetate, cellulose acetate butyrate, cellulose acetate propionate, ethyl cellulose polymers of acrylic and methacrylic acid and esters thereof..

49. The process according to claim 32, wherein the semi-permeable membrane forming polymer is selected from group consisting of cellulose acetate, cellulose acetate butyrate, cellulose acetate propionate, ethyl cellulose, polymers of acrylic and methacrylic acid and esters thereof.

50. The process according to claim 49, wherein the preferred semi-permeable membrane forming polymer is selected from cellulose acetate and ethyl cellulose.

51. The process according to claim 32, wherein the permeable membrane forming polymer is selected from the group consisting of polyvinyl alcohol, polyvinyl pyrrolidone, cellulose ethers, polyethylene glycols, polymers of acrylic and methacrylic acid and esters thereof.

52. The process according to claim 51, wherein the preferred the permeable membrane forming polymer used is selected from polyvinyl pyrrolidone and hydroxypropylmethyl cellulose.

53. The process according to claim 32, wherein the ratio of semi-permeable water insoluble polymer membrane to permeable water soluble polymer membrane is in the range of 9:1 to 1:9, preferably in the range of 9: 1 to 3:7.

54. The process according to claim 32, wherein by adjusting the polymer forming membrane weight coated on the tablet core, thickness of the membrane wall is controlled.

55. The process according to claim 32, wherein the weight of coating solution on the tablet core is up to 20% by weight of tablet core.

56. The process according to claim 32, wherein the thickness of the membrane wall ranges between 1 to 1000 microns, preferably between 50 to 500 microns.

57. The process according to claim 32, wherein the plasticizer used is selected from the group consisting of dibutyl sebacate, diethyl phthalate, dibutyl phthalate, triacetin, triethyl citrate, tributyl citrate, castor oil, propylene glycol, glycerol, polyethylene glycols, liquid sorbitol, and/or mixture thereof.

## Patentansprüche

1. Pharmazeutische Zusammensetzung zur Langzeitfreisetzung einer therapeutisch aktiven Komponente, wobei die Zusammensetzung einen Tablettenkern, der von einer die Freisetzungsgeschwindigkeit kontrollierenden Membran umgeben ist, aufweist, wobei der Tablettenkern besteht aus
(i) der therapeutisch aktiven Komponente mit begrenzter Löslichkeit in den wässerigen Fluiden, die von schwach saurer Natur ist und einen pKa zwischen 2,5 bis 7,5 hat,
(ii) einem alkalisierenden Mittel oder einer Pufferverbindung in unmittelbarem Kontakt mit der obigen therapeutisch aktiven Komponente,
(iii) einer osmotisch wirksamen Beimengung, die in Wasser löslich und in der Lage ist, einen osmotischen Druckgradienten über die die Freisetzungsgeschwindigkeit kontrollierende Membran gegenüber den externen Fluiden auszuüben, und
(iv) gewünschtenfalls einen oder mehrere pharmazeutisch annehmbare Träger und Polymere enthält,
wobei die die Freisetzungsgeschwindigkeit kontrollierende Membran besteht aus:
(i) einem eine semipermeable Membran bildenden Polymer, das in Wasser unlöslich ist, aber für wässerige Fluide teilweise durchlässig und für die Kernzusammensetzung im wesentlichen undurchlässig ist,
(ii) einem eine permeable Membran bildenden Polymer, das in Wasser löslich ist und für wässerige Fluide und mindestens eine der Komponenten der Kernzusammensetzung durchlässig ist; und
(iii) mindestens einem Plastifizierungsmittel im Bereich zwischen 2 bis 60 Gew.% auf der Basis des Gesamtgewichts der trockenen Polymere.

2. Zusammensetzung nach Anspruch 1, bei der das die semipermeable Membran bildende Polymer und das die permeable Membran bildende Polymer zusammen mit Plastifizierungsmittel auf den Tablettenkern aufgetragen und getrocknet sind, um die die Freisetzungsgeschwindigkeit kontrollierende Polymermembran zu erhalten.

3. Zusammensetzung nach Anspruch 1, bei der die therapeutisch aktive Komponente ein Arzneimittel ist, das auf die peripheren Nerven, adrenergen Rezeptoren, cholinergen Rezeptoren, das Nervensystem, die Skelettmuskulatur, die kardiovaskulären, glatten unwillkürlichen Muskeln, das Blutkreislaufsystem, die Synapsenstellen, Neuroeffektor-Verbindungsstellen, das endokrine und das Hormonsystem, das immunologische System, Fortpflanzungssystem, Skelettsystem, die autocoiden Systeme, Verdauungs- und Ausscheidungssysteme, Hemmungs- oder autocoide und Histamin-Systeme wirkt, und solche Materialien sind, die auf das zentrale Nervensystem wirken, wie Schlaf- und Beruhigungsmittel.

4. Zusammensetzung nach Anspruch 1, bei der die therapeutisch aktive Komponente ausgewählt ist aus einer Gruppe, die aus Acetazolamid, Acetylsalicylsäure, p-Aminosalicylsäure, Captropil, Carbenicillin, Carbenoxolon, Chlorpropamid, Clofibrat, Diclofenac, Diflunisal, Ethacrylsäure, Etodolac, Fenoprofen, Furosemid, Gliclazid, Glimepirid, Glipizid, Glyburid, Ibuprofen, Indomethacin, Ketoprofen, Naproxen, Nimesulid, Tolazamid, Tolbutamid, Tolmentin und Zomepirac besteht.

5. Zusammensetzung nach Anspruch 1, bei der die therapeutisch aktive Komponente bevorzugt aus blutzuckersenkenden Mitteln und entzündungshemmenden Mitteln ausgewählt ist.

6. Zusammensetzung nach Anspruch 5, bei der das verwendete blutzuckersenkende Mittel aus der Kategorie Sulfonylharnstoff ausgewählt ist.

7. Zusammensetzung nach Anspruch 6, bei der der verwendete Sulfonylharnstoff ausgewählt ist aus der Gruppe, die aus Glipizid, Gliclazid, Glimepirid und Glyburid besteht.

8. Zusammensetzung nach Anspruch 5, bei der das entzündungshemmende Mittel ausgewählt ist aus der Gruppe, die aus Aspirin, Paracetamol, Ibuprofen, Indomethacin, Ketoprofen, Naproxen, Nimesulid, Tolmentin und Zomepirac besteht, bevorzugt aus Aspirin ausgewählt ist.

9. Zusammensetzung nach Anspruch 1, bei der die Dosis der therapeutisch aktiven Komponente pro Tablette im Bereich zwischen 0,1 mg bis 600 mg liegt.

10. Zusammensetzung nach Anspruch 1, bei der das verwendete alkalisierende Mittel oder der verwendete Puffer in Wasser löslich ist und die Löslichkeit der therapeutisch aktiven Komponente in den wässerigen Fluiden durch Erhöhen des Mikroumgebungs-pH des Kerns über den pKa-Wert der therapeutisch aktiven Komponente verbessert.

11. Zusammensetzung nach Anspruch 10, bei der das verwendete alkalisierende Mittel ausgewählt ist aus der Gruppe, die aus Natrium-bicarbonat, Kalium-bicarbonat, Natrium-citrat, Katium-citrat, Tris(hydroxymethyl)aminomethan, Meglumin und/oder Gemischen davon besteht.

12. Zusammensetzung nach Anspruch 10, bei der der verwendete Puffer ausgewählt ist aus der Gruppe, die aus Natrium-phosphaten, Kalium-phosphaten, Natrium-citrat, Kalium-citrat, Natriumacetat, Tris(hydroxymethyl)-aminomethan und/oder Gemischen davon besteht.

13. Zusammensetzung nach Anspruch 1, bei der das Verhältnis des therapeutisch aktiven Inhaltsstoffs zu dem alkalisierenden Mittel in dem Bereich von 0,1:9,9 bis 7:3 liegt.

14. Zusammensetzung nach Anspruch 1, bei der das Verhältnis des therapeutisch aktiven Inhattsstoffs zu dem Puffer in dem Bereich von 0,1:9,9 bis 7:3 liegt.

15. Zusammensetzung nach Anspruch 1, bei der die verwendete osmotisch wirksame Beimengung ausgewählt ist aus der Gruppe, die aus Natriumchlorid, Kaliumchlorid, Mannitol, Sorbitol und Kohlehydraten, die aus der aus Sucrose, Glucose, Fructose, Dextrose, Lactose bestehenden Gruppe ausgewählt sind, und Gemischen der obigen Osmagenzien besteht.

16. Zusammensetzung nach Anspruch 15, bei der die verwendete osmotisch wirksame Beimengung bevorzugt aus der Gruppe ausgewählt ist, die aus Natriumchlorid, Mannitol und Lactose besteht.

17. Zusammensetzung nach Anspruch 1, bei der die Kernbestandteile einen osmotischen Gradienten über die Wandung der die Freisetzungsgeschwindigkeit kontrollierenden Membran gegenüber den externen Fluiden ausüben.

18. Zusammensetzung nach Anspruch 1, bei der das die semipermeable Membran bildende Polymer wasserunlöslich ist, was es Wasser erlaubt, hindurch zu dringen, und ein Hindurchdringen von Zusammensetzungen des Tablettenkerns im wesentlichen verhindert.

19. Zusammensetzung nach Anspruch 1, bei der das die semipermeable Membran bildende Polymer ausgewählt ist aus der Gruppe, die aus Cellulose-acetat, Cellulose-acetat-buyrat, Cellulose-acetat-propionat, Ethylcellulose, Polymeren von Acrylsäure und Methacrylsäure und Estern davon besteht.

20. Zusammensetzung nach Anspruch 19, bei der das bevorzugte, die semi-permeable Membran bildende Polymer aus Cellulose-acetat und Ethylcellulose ausgewählt ist.

21. Zusammensetzung nach Anspruch 1, bei der das die permeable Membran bildende Polymer wasserlöslich ist, was es Wasser erlaubt, zusammen mit mindestens einem der Bestandteile des Kerns hindurch zu dringen.

22. Zusammensetzung nach Anspruch 1, bei der das die permeable Membran bildende Polymer ausgewählt ist aus der Gruppe, die aus Polyvinylalkohol, Polyvinyl-pyrrolidon, Cellulose-ethern, Polyethylen-glycolen, Polymeren von Acrylsäure und Methacrylsäure und Estern davon besteht.

23. Zusammensetzung nach Anspruch 22, bei der das bevorzugt verwendete, die permeable Membran bildende Polymer aus Polyvinyl-pyrrolidon und Hydroxypropylmethyl-cellulose ausgewählt ist.

24. Zusammensetzung nach Anspruch 1, bei der das Verhältnis von semipermeabler, wasserunlöslicher Polymermembran zu permeabler, wasserlöslicher Polymermembran in den Bereich von 9:1 bis 1:9, bevorzugt in dem Bereich von 9:1 bis 3:7, liegt.

25. Zusammensetzung nach Anspruch 1, bei der das verwendete Plastifizierungsmittel kontrollierte Löslichkeit in Wasser hat.

26. Zusammensetzung nach Anspruch 25, bei das verwendete Plastifizierungsmittel ausgewählt ist aus der Gruppe, die aus Dibutyl-sebacat, Diethylphthalat, Dibutyl-phthalat, Triacetin, Triethyl-citrat, Tributyl-citrat, Castoröl, Propylen-glycol, Glycerol, Polyethylen-glycolen, flüssigem Sorbitol und/oder Gemischen davon besteht.

27. Zusammensetzung nach Anspruch 1, bei der die Dicke der Membranwandung durch Einstellen des auf den Tablettenkern aufgetragenen Gewichts des die Membran bildenden Polymers kontrolliert wird.

28. Zusammensetzung nach Anspruch 1, bei der die Dicke der Membranwandung zwischen 1 bis 1000µm liegt.

29. Zusammensetzung nach Anspruch 28, bei der die bevorzugte Dicke der Membranwandung im Bereich zwischen 50 bis 500 µm liegt.

30. Zusammensetzung nach Anspruch 1, bei der der verwendete pharmazeutisch annehmbare Träger/die verwendeten pharmazeutisch annehmbaren Träger ausgewählt ist (sind) aus der Gruppe, die aus Magnesium-stearat, Talk und Aerosil besteht.

31. Zusammensetzung nach Anspruch 1, wobei der Freisetzungsmechanismus des therapeutisch aktiven Inhaltsstoffs daraus auf der Kombination von osmotischem Pumpen und Diffusion basiert.

32. Verfahren zur Herstellung einer pharmazeutischen Zusammensetzung, wie sie in Anspruch 1 beansprucht ist, zur Langzeitfreisetzung einer therapeutisch aktiven Komponente, wobei das Verfahren aufweist:
a. Herstellen einer Kernzusammensetzung durch Trockenvermischen einer therapeutisch aktiven Komponente, eines alkalisierenden Mittels oder einer Pufferverbindung, einer osmotisch wirksamen Beimengung und gewünschtenfalls einen oder mehrere pharmazeutisch annehmbare Träger und Polymere enthaltend, oder
b. Herstellen der Kernzusammensetzung durch Stoß- oder Feuchtgranulierungs-Techniken durch Vermischen des Arzneimittels mit den anderen Trägern einschließlich alkalisierendem Mittel (alkalisierenden Mitteln)/Puffer(n) und Osmagens unter Verwendung von Wasser, Alkohol oder eines organischen Verschnittmittels wie Isopropylalkohol/Methylenchlorid, im Verhältnis 80/20 VN, als das Granulierungsfluid, um feuchte Granalien zu erhalten, oder
c. durch Lösen des Arzneimittels und des Löslichkeits-Modifikationsmittels in einem gemeinsamen wässerigen oder organischen Lösungsmittel und danach Eindampfen zur Trockne. Mischen des Rückstands mit Osmagens und anderen zur Erhaltung der Kernzusammensetzung erforderlichen Trägern,
d. Zusammenpressen der obigen, in den Schritten (a), (b) und (c) erhaltenen Kernzusammensetzung unter Verwendung einer konventionellen Tablettiermaschine, um Tablettenkerne zu erhalten,
e. Herstellen einer Beschichtungslösung durch Auflösen der erforderlichen Menge an die semipermeable Membran bildendem Polymer in einem Lösungsmittel, das aus Methylenchlorid, Methanol, Ethanol oder Gemischen davon ausgewählt wird,
f. Hinzufügen von die permeable Membran bildendem Polymer und von einem oder mehreren Plastifizierungsmitteln zu der Lösung von Schritt (e) unter dauerndem Rühren,
g. Beschichten der zusammengepreßten Tablette aus Schritt (d) mit der Beschichtungslösung aus Schritt (f) unter Verwendung der Techniken, die ausgewählt werden aus Preßbeschichtungs-, Sprüh-, Tauch- oder Luftsuspensions-Techniken, und
h. Trocknen des in Schritt (g) erhaltenen, beschichteten Tablettenkerns bei 45 - 60° C für etwa 16 Stunden, um die Zusammensetzung für Langzeitfreisetzung zu erhalten, und Verpacken der Tabletten durch konventionelle Verfahren.

33. Verfahren nach Anspruch 32, bei dem in Schritt (a) das durch Trockenvermischen erhaltene Gemisch durch Standardsiebe passiert wird, um eine gleichmäßige Teilchengröße zu erhalten, um die Kernzusammensetzung zu bilden.

34. Verfahren nach Anspruch 32, bei dem in Schritt (b) die feuchten Granalien bei einer Temperatur im Bereich zwischen 40 und 60° C etwa 10 Minuten lang getrocknet werden, die Granalien durch Standardsiebe 20 - 22 passiert werden, um Agglomerate aufzubrechen und zu einer gleichmäßigen Teilchengröße zu machen, um eine Kernzusammensetzung zu erhalten.

35. Verfahren nach Anspruch 32, bei dem die therapeutisch aktive Komponente ausgewählt wird aus einer Gruppe, die aus Acetazolamid, Acetylsalicylsäure, p-Aminosalicylsäure, Captropil, Carbenicillin, Carbenoxolon, Chlorpropamid, Clofibrat, Diclofenac, Diflunisal, Ethacrylsäure, Etodolac, Fenoprofen, Furosemid, Gliclazid, Glimepirid, Glipizid, Glyburid, Ibuprofen, Indomethacin, Ketoprofen, Naproxen, Nimesulid, Tolazamid, Tolbutamid, Tolmentin und Zomepirac besteht.

36. Verfahren nach Anspruch 32, bei dem die therapeutisch aktive Komponente bevorzugt aus blutzuckersenkenden Mitteln und entzündungshemmenden Mitteln ausgewählt wird,

37. Verfahren nach Anspruch 36, bei dem das verwendete blutzuckersenkende Mittel aus der Kategorie Sulfonylharnstoff ausgewählt wird.

38. Verfahren nach Anspruch 37, bei dem der verwendete Sulfonylharnstoff ausgewählt wird aus der Gruppe, die aus Glipizid, Gliclazid, Glimepirid und Glyburid besteht.

39. Verfahren nach Anspruch 36, bei dem das entzündungshemmende Mittel ausgewählt wird aus der Gruppe, die aus Aspirin, Paracetamol, Ibuprofen, Indomethacin, Ketoprofen, Naproxen, Nimesulid, Tolmentin und Zomepirac besteht, bevorzugt aus Aspirin ausgewählt wird.

40. Verfahren nach Anspruch 32, bei dem die Dosis der therapeutisch aktiven Komponente pro Tablette im Bereich zwischen 0,1 mg bis 600 mg liegt.

41. Verfahren nach Anspruch 32, bei dem das verwendete alkalisierende Mittel ausgewählt wird aus der Gruppe, die aus Natrium-bicarbonat, Kalium-bicarbonat, Natrium-citrat, Kalium-citrat, Tris(hydroxymethyl)aminomethan, Meglumin und/oder den Gemischen davon besteht.

42. Verfahren nach Anspruch 32, bei dem der verwendete Puffer ausgewählt wird aus der Gruppe, die aus Natrium-phosphaten, Kalium-phosphaten, Natrium-citrat, Kalium-citrat, Natrium-acetat, Tris/hydroxymethyl)aminomethan und/ oder Gemischen davon besteht.

43. Verfahren nach Anspruch 32, bei dem das Verhältnis des therapeutisch aktiven Inhaltsstoffs zu dem alkalisierenden Mittel in dem Bereich von 0,1:9,9 bis 7:3 liegt.

44. Verfahren nach Anspruch 32, bei dem das Verhältnis des therapeutisch aktiven Inhaltsstoffs zu dem Puffer in dem Bereich von 0,1:9,9 bis 7:3 liegt.

45. Verfahren nach Anspruch 32, bei dem die verwendete osmotisch wirksame Beimengung ausgewählt wird aus der Gruppe, die aus Natriumchlorid, Kaliumchlorid, Mannitol, Sorbitol und Kohlehydraten, die aus der aus Sucrose, Glucose, Fructose, Dextrose, Lactose bestehenden Gruppe ausgewählt sind, und Gemischen davon besteht.

46. Verfahren nach Anspruch 45, bei dem die verwendete osmotisch wirksame Beimengung bevorzugt ausgewählt wird aus der Gruppe, die aus Natriumchlorid, Mannitol und Lactose besteht.

47. Verfahren nach Anspruch 32, bei dem der verwendete Träger (die verwendeten Träger) ausgewählt wird (werden) aus der Gruppe, die aus Magnesium-stearat, Talk und Aerosil besteht.

48. Verfahren nach Anspruch 32, bei dem das verwendete Polymer (die verwendeten Polymere) ausgewählt wird (werden) aus der Gruppe, die aus Polyvinylalkohol, Polyvinyl-pyrrolidon, Cellulose-ethern, Polyethylen-glycolen, Cellulose-acetat, Cellulose-acetat-butyrat, Cellulose-acetat-propionat, Ethylcellulose-Polymeren von Acrylsäure und Methacrylsäure und Estern davon besteht.

49. Verfahren nach Anspruch 32, bei dem das die semipermeable Membran bildende Polymer ausgewählt wird aus der Gruppe, die aus Cellulose-acetat, Cellulose-acetat-butyrat, Cellulose-acetat-propionat, Ethylcellulose, Polymeren von Acrylsäure und Methacrylsäure und Estern davon besteht.

50. Verfahren nach Anspruch 49, bei dem das bevorzugte, die semipermeable Membran bildende Polymer ausgewählt wird aus Cellulose-acetat und Ethylcellulose.

51. Verfahren nach Anspruch 32, bei dem das die permeable Membran bildende Polymer ausgewählt wird aus der Gruppe, die aus Polyvinylalkohol, Polyvinyl-pyrrolidon, Cellulose-ethern, Polyethylen-glycolen, Polymeren von Acrylsäure und Methacrylsäure und Estern davon besteht.

52. Verfahren nach Anspruch 51, bei dem das bevorzugt verwendete, die permeable Membran bildende Polymer ausgewählt wird aus Polyvinyl-pyrrolidon und Hydroxypropylmethyl-cellulose.

53. Verfahren nach Anspruch 32, bei dem das Verhältnis von semipermeabler, wasserunlöslicher Polymermembran zu permeabler, wasserlöslicher Polymermembran in dem Bereich von 9:1 bis 1:9, bevorzugt in dem Bereich von 9:1 bis 3:7, liegt.

54. Verfahren nach Anspruch 32, bei dem die Dicke der Membranwandung durch Einstellen des auf den Tablettenkern aufgetragenen Gewichts des die Membran bildenden Polymers kontrolliert wird.

55. Verfahren nach Anspruch 32, bei dem das Gewicht der Beschichtungslösung auf dem Tablettenkern bis zu 20 Gew.% des Tablettenkerns beträgt.

56. Verfahren nach Anspruch 32, bei dem die Dicke der Membranwandung in dem Bereich zwischen 1 bis 1000 µm, bevorzugt zwischen 50 bis 500 µm, liegt.

57. Verfahren nach Anspruch 32, bei dem das verwendete Plastifizierungsmittel ausgewählt wird aus der Gruppe, die aus Dibutyl-sebacat, Diethylphthalat, Dibutyl-phthalat, Triacetin, Triethyl-citrat, Tributyl-citrat, Castoröl, Propylen-glycol, Glycerol, Polyethylen-glycolen, flüssigem Sorbitol und/oder Gemischen davon besteht.

## Revendications

1. Composition pharmaceutique pour la libération prolongée d'une entité active sur le plan thérapeutique, ladite composition consistant en un noyau de comprimé entouré d'une membrane contrôlant la vitesse de libération, ledit noyau de comprimé consistant en :
(i) une entité active sur le plan thérapeutique ayant une solubilité limitée dans les fluides aqueux, de nature faiblement acide et ayant un pKa entre 2,5 et 7,5,
(ii) un agent alcalinisant ou un composé tampon en contact direct avec ladite entité active sur le plan thérapeutique ci-dessus ;
(iii) un soluté efficace du point de vue osmotique qui est soluble dans l'eau et capable de présenter un gradient de pression osmotique à travers la membrane contrôlant la vitesse de libération contre les fluides externes, et
(iv) éventuellement contenant un ou plusieurs excipients et polymères acceptables sur le plan pharmaceutique,
ladite membrane contrôlant la vitesse de libération consistant en :
(i) un polymère formant membrane semi-perméable qui est insoluble dans l'eau mais partiellement perméable aux fluides aqueux et sensiblement imperméable à la composition de noyau ;
(ii) un polymère formant membrane perméable qui est soluble dans l'eau et perméable aux fluides aqueux et à au moins l'une des entités de la composition de noyau ; et
(iii) au moins un plastifiant représentant entre 2 et 60 % en poids du poids total des polymères secs.

2. La composition selon la revendication 1, dans laquelle le polymère formant membrane semi-perméable et le polymère formant membrane perméable, avec le plastifiant, enrobent le noyau de comprimé et sont séchés pour donner la membrane polymère contrôlant la vitesse de libération.

3. La composition selon la revendication 1 dans laquelle le groupement actif sur le plan thérapeutique est un médicament qui agit sur les nerfs périphériques, les récepteurs adrénergiques, les récepteurs cholinergiques, le système nerveux, les muscles du squelette, le système cardio-vasculaire, les muscles lisses, le système circulatoire, les sites synaptiques, les sites de jonction neuroeffectrice, le système hormonal et endocrinien, le système immunologique, le système reproducteur, le système squelettique, les systèmes autocoïdes, les systèmes alimentaire et excréteur, les systèmes inhibiteurs ou autocoïdes et histaminiques, et les substances agissant sur le système nerveux central comme les hypnotiques et les sédatifs.

4. La composition selon la revendication 1, dans laquelle l'entité active sur le plan thérapeutique est choisie dans le groupe consistant en l'acétazolamine, l'acide acétylsalicylique, l'acide p-aminosalicylique, le captropil, la carbénicilline, le carbénoxolone, le chlorpropamide, le clofibrate, le diclofénac, le diflunisal, l'acide éthacrynique, l'étodolac, le fénoprofène, le furosémide, le gliclazide, le glimépiride, le glipizide, le glyburide, l'ibuprofène, l'indométacine, le kétoprofène, le naproxène, le nimésulide, la tolazamide, le tolbutamide, le tolmentin et le zomépirac.

5. La composition selon la revendication 1, dans laquelle l'entité active sur le plan thérapeutique est de préférence choisie parmi un agent hypoglycémiant et un agent anti-inflammatoire.

6. La composition selon la revendication 5, dans lequel l'agent hypoglycémiant utilisé est choisi dans la catégorie des sulfonylurées.

7. La composition selon la revendication 6, dans laquelle la sulfonylurée utilisée est choisie dans le groupe consistant en le glipizide, le gliclazide, le glimépiride et le glyburide.

8. La composition selon la revendication 5, dans laquelle l'agent anti-inflammatoire est choisi dans le groupe consistant en l'aspirine, le paracétamol, l'ibuprofène, l'indométacine, le kétoprofène, le naproxène, le nimésulide, le tolmentin et le zomépirac, de préférence choisi parmi Asprin.

9. La composition selon la revendication 1, dans laquelle la dose de l'entité active sur le plan thérapeutique par comprimé est de 0,1 mg à 600 mg.

10. La composition selon la revendication 1, dans laquelle l'agent alcalinisant ou le tampon sont solubles dans l'eau et améliorent la solubilité de l'entité active sur le plan thérapeutique dans les fluides aqueux en augmentant le pH micro-environnemental du noyau au-dessus de la valeur de pKa du groupement actif sur le plan thérapeutique.

11. La composition selon la revendication 10, dans laquelle l'agent alcalinisant utilisé est choisi dans le groupe consistant en le bicarbonate de sodium, le bicarbonate de potassium, le citrate de sodium, le citrate de potassium, les tris(hydroxyméthyl)aminométhane, le méglumine, et/ou leur mélange.

12. La composition selon la revendication 10, dans laquelle le tampon utilisé est choisi dans le groupe consistant en les phosphates de sodium, les phosphates de potassium, le citrate de sodium, le citrate de potassium, l'acétate de sodium, le tris(hydroxyméthyl)aminométhane et/ou leurs mélanges.

13. Une composition selon la revendication 1, dans laquelle le ratio de l'ingrédient actif sur le plan thérapeutique à l'agent alcalinisant est dans la plage de 0,1/9,9 à 7/3.

14. La composition selon la revendication 1, dans laquelle le rapport de l'ingrédient actif sur le plan thérapeutique tampon est dans la plage de 0,1/9,9 à 7/3.

15. Une composition selon la revendication 1, dans laquelle le soluté efficace sur le plan osmotique utilisé est choisi dans le groupe consistant en le chlorure de sodium, le chlorure de potassium, le mannitol, le sorbitol, et des hydrates de carbone choisis dans le groupe consistant en le sucrose, le glucose, le fructose, le dextrose, le lactose et les mélanges des agents osmotiques ci-dessus.

16. Une composition selon la revendication 15, dans laquelle le soluté efficace sur le plan osmotique utilisé est de préférence choisi dans le groupe consistant en le chlorure de sodium, le mannitol et le lactose.

17. Une composition selon la revendication 1, dans laquelle les composants du noyau exercent un gradient osmotique à travers la paroi de membrane contrôlant la vitesse de libération contre les fluides externes.

18. Une composition selon la revendication 1, dans laquelle le polymère formant membrane semi-perméable est insoluble dans l'eau, ce qui permet à l'eau de passer et empêche sensiblement la perméabilité des compositions du noyau de comprimé.

19. Une composition selon la revendication 1, dans laquelle le polymère formant membrane semi-perméable est choisi dans le groupe consistant en l'acétate de cellulose, l'acétobutyrate de cellulose, l'acétopropionate de cellulose, l'éthylcellulose, des polymères d'acide acrylique et méthacrylique et leurs esters.

20. Une composition selon la revendication 19, dans laquelle le polymère formant membrane semi-perméable préféré est choisi parmi l'acétate de cellulose et l'éthylcellulose.

21. Une composition selon la revendication 1, dans laquelle le polymère formant membrane perméable est hydrosoluble, ce qui permet à l'eau de pénétrer avec au moins l'un des composants du noyau.

22. Une composition selon la revendication 1, dans laquelle le polymère formant membrane perméable est choisi dans le groupe consistant en l'alcool polyvinylique, la polyvinylpyrrolidone, les éthers de cellulose, les polyéthylène glycols, les polymères d'acide acrylique et méthacrylique et leurs esters.

23. Une composition selon la revendication 22, dans laquelle le polymère formant membrane perméable préféré est choisi parmi la polyvinylpyrrolidone et l'hydroxypropylméthylcellulose.

24. Une composition selon la revendication 1, dans laquelle le rapport de membrane de polymère insoluble dans l'eau semi-perméable sur la membrane polymère hydrosoluble perméable est dans la plage de 9/1 à 1/9, de préférence dans la plage de 9/1 à 3/7.

25. Une composition selon la revendication 1, dans laquelle le plastifiant utilisé a une solubilité contrôlée dans l'eau

26. Une composition selon la revendication 25, dans laquelle le plastifiant utilisé est choisi dans le groupe consistant en le sébaçate de dibutyle, le phtalate de diéthyle, le phtalate de dibutyle, la triacétine, le citrate de triéthyle, le citrate de tributyle, l'huile de ricin, le propylène glycol, le glycérol, les polyéthylène glycols, le sorbitol liquide et/ou leurs mélanges.

27. Composition selon la revendication 1, dans laquelle, en ajustant le poids de polymère formant la membrane revêtant le noyau du comprimé, l'épaisseur de la paroi de la membrane est contrôlée.

28. Une composition selon la revendication 1, dans laquelle l'épaisseur de la paroi de la membrane est comprise entre 1 et 1 000 microns.

29. Une composition selon la revendication 28, dans laquelle l'épaisseur préférée de la paroi de la membrane est comprise entre 50 et 500 microns.

30. Une composition selon la revendication 1, dans laquelle le ou les excipients pharmaceutiquement acceptables utilisés est ou sont choisis dans le groupe consistant en le stéarate de magnésium, le talc et l'aérosil.

31. Composition selon la revendication 1 dont le mécanisme de libération de l'ingrédient actif sur le plan thérapeutique est fondé sur la combinaison du pompage osmotique et de la diffusion.

32. Un procédé pour préparer une composition pharmaceutique selon la revendication 1 pour la libération prolongée d'une entité active sur le plan thérapeutique, ledit procédé consistant en les étapes de:
a. préparation d'une composition de noyau par mélange à sec d'une entité active sur le plan thérapeutique, d'un agent alcalinisant ou d'un composé tampon, d'un soluté efficace sur le plan osmotique et facultativement contenant un ou plusieurs excipients ou polymères pharmaceutiquement acceptables, ou
b. préparation de la composition de noyau par des techniques de compression du noyau ou de granulation humide ou en suspension en mélangeant le médicament avec les autres excipients dont le ou les agents alcalinisants/le ou les tampons et l'agent osmotique en utilisant de l'eau, de l'alcool ou un cosolvant organique comme de l'alcool isopropylique/ chlorure de méthylène, selon un rapport de 80/20, V/V comme fluide de granulation pour obtenir des granules humides, ou
c. dissolution du médicament et du modificateur de solubilité dans un solvant aqueux ou organique courant et après évaporation à sec, malaxage du résidu avec l'agent osmotique ou d'autres excipients nécessaires pour obtenir la composition de noyau,
d. compression de la composition de noyau ci-dessus obtenu aux étapes (a), (b) et (c) pour obtenir un noyau de comprimé en utilisant une machine à comprimés classique,
e. préparation d'une solution de revêtement en dissolvant la quantité requise de polymère formant membrane semi-perméable dans un solvant choisi parmi le chlorure de méthylène, le méthanol, l'éthanol ou leurs mélanges,
f. addition de polymère formant membrane perméable et d'un ou de plusieurs plastifiants à la solution de l'étape (e) sous agitation continue,
g. enrobage du comprimé de l'étape (d) avec la solution de revêtement de l'étape (f) en utilisant les techniques choisies parmi l'enrobage à sec, la pulvérisation, l'immersion ou la pulvérisation sur comprimés en suspension dans l'air et
h. séchage du noyau de comprimé enrobé obtenu à l'étape (g) à 45-60 °C pendant environ 16 heures pour obtenir la composition pour libération prolongée et emballage des comprimés par des procédés classiques.

33. Un procédé selon la revendication 32 dans lequel à l'étape (a), le mélange obtenu par mélange à sec est passé sur des tamis normalisés pour obtenir une taille de particules uniformes de manière à former la composition de noyau.

34. Le procédé selon la revendication 32 dans lequel à l'étape (b) les granules humides sont séchés à une température allant entre 40 et 60 °C pendant environ 10 minutes, puis passent sur un tamis normalisé 20-22 pour rompre les agglomérats et pour leur donner une taille de particule uniforme pour obtenir la composition de noyau.

35. Le procédé selon la revendication 32, dans lequel l'entité active sur le plan thérapeutique est choisie dans un groupe consistant en l'acétazolamide, l'acide acétylsalicylique, l'acide p-aminosalicylique, le captropil, la carbénicilline, le carbénoxolone, le chlorpropamide, le clofibrate, le diclofénac, le diflunisal, l'acide éthacrynique, l'étodolac, le fénoprofène, le furosémide, le gliclazide, le glimépiride, le glipizide, le glyburide, l'ibuprofène, l'indométacine, le kétoprofène, le naproxène, le nimésulide, la tolazamide, le tolbutamide, le tolmentin et le zomépirac.

36. Le procédé selon la revendication 32, dans lequel l'entité active sur le plan thérapeutique est de préférence choisie parmi un agent hypoglycémiant et un agent anti-inflammatoire.

37. Le procédé selon la revendication 36, dans lequel l'agent hypoglycémiant utilisé est choisi dans la catégorie des sulfonylurées.

38. Le procédé selon la revendication 37, dans lequel la sulfonylurée utilisée est choisie dans le groupe consistant en le glipizide, le gliclazide, le glimépiride, et le glyburide.

39. Le procédé selon la revendication 36, dans lequel l'agent anti-inflammatoire est choisi dans le groupe consistant en l'aspirine, le paracétamol, l'ibuprofene, l'indométacine, le kétoprofène, le naproxène, le nimésulide, le tolmentin et le zomépriac, de préférence choisi parmi Asprin.

40. Le procédé selon la revendication 32, dans lequel la dose d'entité active sur le plan thérapeutique par comprimé est comprise entre 0,1 et 600 mg.

41. Le procédé selon la revendication 32, dans lequel l'agent alcalinisant utilisé est choisi dans le groupe consistant en le bicarbonate de sodium, le bicarbonate de potassium, le citrate de sodium, le citrate de potassium, le tris(hydroxyméthyl)aminométhane, le méglumine, et/ou leur mélange.

42. Le procédé selon la revendication 32, dans lequel le tampon utilisé est choisi dans le groupe consistant en les phosphates de sodium, les phosphates de potassium, le citrate de sodium, le citrate de potassium, l'acétate de sodium, le tris(hydroxyméthyl)aminométhane et/ou leurs mélanges.

43. Le procédé selon la revendication 32, dans lequel rapport de l'ingrédient actif sur le plan thérapeutique à l'agent alcalinisant est dans la plage de 0,1/9,9 à 7/3.

44. Le procédé selon la revendication 32, dans lequel le rapport de l'ingrédient actif sur le plan thérapeutique au tampon est dans la plage de 0,1/9,9 à 7/3.

45. Le procédé selon la revendication 32, dans lequel le soluté efficace sur le plan osmotique utilisé est choisi dans le groupe consistant en le chlorure de sodium, le chlorure de potassium, le mannitol, le sorbitol, et des hydrates de carbone choisis dans le groupe consistant en la saccharose, le glucose, le fructose, le dextrose, le lactose et leurs mélanges.

46. Le procédé selon la revendication 45, dans lequel le soluté efficace sur le plan osmotique utilisé est de préférence choisi dans le groupe consistant en le chlorure de sodium, le mannitol et le lactose.

47. Le procédé selon la revendication 32, dans lequel le ou les excipients utilisés sont choisis dans le groupe consistant en le stéarate de magnésium, le talc et l'aérosil.

48. Le procédé selon la revendication 32, dans lequel le ou les polymères utilisés sont choisis dans le groupe consistant en l'alcool polyvinylique, la polyvinylpyrrolidone, les éthers de cellulose, les polyéthylène glycols, l'acétate de cellulose, l'acétobutyrate de cellulose, l'acétopropionate de cellulose, des polymères d'éthylcellulose d'acide acrylique et méthacrylique et leurs esters.

49. Le procédé selon la revendication 32, dans lequel le polymère formant membrane semi-perméable est choisi dans le groupe consistant en l'acétate de cellulose, l'acétobutyrate de cellulose, l'acétopropionate de cellulose, l'éthylcellulose, des polymères d'acide acrylique et méthacrylique et leurs esters.

50. Le procédé selon la revendication 49, dans lequel le polymère formant membrane semi-perméable préféré est choisi parmi l'acétate de cellulose et l'éthylcellulose.

51. Le procédé selon la revendication 32, dans lequel le polymère formant membrane perméable est choisi dans le groupe consistant en l'alcool polyvinylique, la polyvinylpyrrolidone, les éthers de cellulose, les polyéthylène glycols, les polymères d'acide acrylique et méthacrylique et leurs esters.

52. Le procédé selon la revendication 51, dans lequel le polymère formant membrane perméable préféré est choisi parmi la polyvinylpyrrolidone et l'hydroxypropylméthylcellulose.

53. Le procédé selon la revendication 32, dans lequel le rapport de la membrane de polymère insoluble dans l'eau semi-perméable sur la membrane polymère hydrosoluble perméable est dans la plage de 9/1 à 1/9, de préférence dans la plage de 9/1 à 3/7.

54. Le procédé selon la revendication 32, dans lequel en ajustant le poids de polymère formant la membrane revêtant le noyau du comprimé, l'épaisseur de la paroi de la membrane est contrôlée.

55. Le procédé selon la revendication 32, dans lequel le poids de solution de revêtement sur le noyau de comprimé est au maximum de 20 % du poids du noyau de comprimé.

56. Le procédé selon la revendication 32, dans lequel l'épaisseur de la paroi de la membrane est comprise entre 1 et 1 000 microns, de préférence entre 50 et 500 microns.

57. Le procédé selon la revendication 32, dans lequel le plastifiant utilisé est choisi dans le groupe consistant en le sébaçate de dibutyle, le phtalate de diéthyle, le phtalate de dibutyle, la triacétine, le citrate de triéthyle, le citrate de tributyle, l'huile de ricin, le propylène glycol, le glycérol, les polyéthylène glycols, le sorbitol liquide et/ou leurs mélanges.
